(19) Europäisches Patentamt

European Patent Office

Office européen des brevets

(11) **EP 4 258 205 B1**

(12) **EUROPEAN PATENT SPECIFICATION**

(45) Date of publication and mention of the grant of the patent:
**17.12.2025 Bulletin 2025/51**

(21) Application number: **21912791.7**

(22) Date of filing: **29.04.2021**

(51) International Patent Classification (IPC):
**G06T 7/00** *(2017.01)*    **A61B 3/00** *(2006.01)*
**A61B 3/12** *(2006.01)*    **G16H 30/40** *(2018.01)*

(52) Cooperative Patent Classification (CPC):
**G16H 30/40; A61B 3/0025; G06T 7/0012;**
**G06T 7/0014**

(86) International application number:
**PCT/CN2021/091225**

(87) International publication number:
**WO 2022/142029 (07.07.2022 Gazette 2022/27)**

(54) **QUALITY CONTROL METHOD AND QUALITY CONTROL SYSTEM FOR DATA ANNOTATION ON FUNDUS IMAGE**

QUALITÄTSKONTROLLVERFAHREN UND QUALITÄTSKONTROLLSYSTEM FÜR DATENANNOTATION AUF FUNDUSBILD

PROCÉDÉ DE CONTRÔLE DE QUALITÉ ET SYSTÈME DE CONTRÔLE DE QUALITÉ POUR UNE ANNOTATION DE DONNÉES SUR UNE IMAGE DE FOND D'OEIL

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.12.2020  CN 202011588182**

(43) Date of publication of application:
**11.10.2023  Bulletin 2023/41**

(73) Proprietor: **Shenzhen Sibright Technology Co., Ltd.**
**Baoan District,**
**Shenzhen,**
**Guangdong 518000 (CN)**

(72) Inventors:
• **WANG, Juan**
**Shenzhen, Guangdong 518000 (CN)**

• **HU, Juan**
**Shenzhen, Guangdong 518000 (CN)**
• **HU, Zhigang**
**Shenzhen, Guangdong 518000 (CN)**
• **LAI, Ming**
**Shenzhen, Guangdong 518000 (CN)**

(74) Representative: **Isern Patentes y Marcas S.L.**
**Avda. Diagonal, 463 Bis, 2°**
**08036 Barcelona (ES)**

(56) References cited:
**WO-A1-2020/081075      CN-A- 109 166 126**
**CN-A- 110 991 486      CN-A- 111 063 422**
**CN-A- 111 080 577      US-A1- 2018 165 850**
**US-A1- 2020 152 316**

**Description**

**BACKGROUND OF INVENTION**

**Field of Invention**

The present disclosure relates to a quality control method and a quality control system for data annotation on a fundus image.

**Description of Related Art**

With the development of artificial intelligence technology, supervised learning technology based on machine learning has been applied in more and more fields.Especially in the field of medical imaging, supervised learning technology based on machine learning is a big success. In supervised learning, a machine learning model is trained using a training set consisting of training data (e.g., fundus images) and annotation results of the training data (e.g., diabetic retinopathy staging), so the data annotation quality of the training data is crucial to the training of the model.

Currently, in order to make the annotation result of training data to be more accurate, professional annotators such as professional ophthalmologists are often allowed to annotate training data and perform quality control on the annotation result in combination with quality control methods. For example, literature (CN110991486 A) discloses a method for multi-person collaborative image annotation quality control, in which gold-standard data is input into an annotation package according to a pre-set proportion so as to verify the annotation quality of any annotation package annotated by an annotation user. In a multi-person fitting step, an image is distributed to a plurality of users, annotation results of the image by the plurality of users are collected, and a real label is obtained after repeated labels are obtained. However, the accuracy of the annotation results of the training data needs to be improved. US2018165850A1 discloses a computer-implemented method for automatic generation of fundus drawings, wherein fundus images undergo image processing to extract features using deep learning-based analysis and other techniques. The method assigns classifiers to identified features and applies machine learning models to generate recommended labels for fundus drawings. CN111080577A discloses a fundus image quality assessment method that acquires captured images, discriminates whether they are fundus images, performs color conversion and channel separation to obtain grayscale images, and extracts global low-quality pixel distribution areas. The method evaluates image quality by analyzing low-quality pixels in restricted areas and provides corresponding quality assessment results with causes for low-quality images.

**SUMMARY OF INVENTION**

The present disclosure has been made in view of the above-mentioned circumstances, and it is an object of the present disclosure to provide a quality control method and a quality control system for data annotation on a fundus image with high accuracy. The invention is set out in the appended set of claims.

For the above purpose, a first aspect of the present disclosure provides a quality control method for data annotation on a fundus image, including: acquiring a plurality of fundus images; performing standardization processing on each of the plurality of fundus images to obtain a plurality of standardized fundus images; performing preliminary filtering on quality of each of the plurality of standardized fundus images to obtain a plurality of qualified fundus images; preparing a target fundus image set, the target fundus image set includes a data set to be calibrated including the plurality of qualified fundus images, a gold-standard data set including a first preset number of gold-standard fundus images with a known correct annotation result, and a self-consistency determination data set composed of at least one image in the data set to be calibrated, and taking each image of the target fundus image set as a respective target fundus image;annotating respective images of the target fundus image set by a plurality of first annotation doctors respectively to obtain a plurality of groups of doctor annotation results, the doctor annotation results include at least one determination result, the determination result at least includes disease information of no obvious abnormality or of a disease; calculating self-consistency and gold-standard consistency of the corresponding first annotation doctors on the basis of the doctor annotation results to acquire the doctor annotation results of the first annotation doctors satisfying a preset condition as target annotation results, obtaining the self-consistency by taking any one of two groups of annotation results of the doctor annotation result of each image in the self-consistency determination data set and the doctor annotation result of an image, which is repeated with respective image in the self-consistency determination data set, in the data set to be calibrated as a first group of annotation results and taking the other group as a second group of annotation results and performing evaluation using a self-consistency determination and evaluation method, acquiring the gold-standard consistency by taking the correct annotation result of the gold-standard data set as a first group of annotation results and the doctor annotation result of each image in the gold-standard data set as a second group of annotation results and using a gold-standard consistency determination and evaluation method;gathering a plurality of sets of the target annotation results to obtain a final annotation result. In this case, on the basis of the gold-standard data set and the self-consistency determination data set, the doctor annotation result of the first annotation doctor that satisfies the preset conditions can be obtained as the target annotation result and gathered. Thus, the accuracy of data annotation of the fundus image can be improved.

In addition, in the quality control method according to the first aspect of the present disclosure, optionally, the preset condition is that the self-consistency is greater than a self-consistency threshold value and the gold-standard consistency is greater than a gold-standard consistency threshold value. Thus, the preset condition can be determined based on the self-consistency threshold value and the gold-standard consistency threshold value.

In addition, in the quality control method according to the first aspect of the present disclosure, optionally, when the doctor annotation result of the first annotation doctor does not meet the preset condition, each image in the target fundus image set is re-annotated by the second annotation doctor until the doctor annotation result meeting the preset condition is obtained as the target annotation result. Thus, a target annotation result can be obtained.

In addition, in the quality control method according to the first aspect of the present disclosure, optionally, the self-consistency determination method is to calculate a disease self-consistency of each of the disease determined by each first annotation doctor using a quadratic weighted kappa coefficient and to weight each disease self-consistency so as to calculate the self-consistency of each first annotation doctor; the gold-standard consistency determination method is to calculate the gold-standard consistency of each of the disease determined by each first annotation doctor using a quadratic weighted kappa coefficient and to weight the gold-standard consistency of the disease so as to calculate the gold-standard consistency of each first annotation doctor. Thus, the self-consistency of each first annotation doctor can be calculated based on the self-consistency determination method and the gold-standard consistency of each first annotation doctor can be calculated based on the gold-standard consistency determination method.

In addition, in the quality control method according to the first aspect of the present disclosure, optionally, the quadratic weighted kappa coefficient $\kappa$ is

$$\kappa = 1 - \frac{\sum_{i,j} W_{ij} X_{ij}}{\sum_{i,j} W_{ij} E_{ij}}$$

, wherein $W_{ij}$ represents a quadratic weighting coefficient, $X_{ij}$ represents a number of the target fundus images for which the determination result in the first group of annotation results is i and the determination result in the second group of annotation results is *j*, and $E_{ij}$ represents an expected number of the target fundus images for which the determination result in the first group of annotation results is *i* and the determination result in the second group of annotation results is *j*. Thus, it is able to inspect consistency between the first group of annotation results and the second group of annotation results.

In addition, in the quality control method according to the first aspect of the present disclosure, optionally, the self-consistency threshold value and the gold-standard consistency threshold value are determined through analyzing target self-consistency and target gold-standard consistency of doctors with different threshold value annotationusing abnormality detection. Thus, the self-consistency threshold value and the gold-standard consistency threshold value can be determined.

In addition, in the quality control method according to the first aspect of the present disclosure, optionally, the abnormality detection is to acquire the target self-consistency of the doctors with different threshold value annotation and calculate a self-consistency mean value $\mu_0$ and a self-consistency variance $\sigma_0$, under the assumption that the target self-consistency satisfies a Gaussian distribution, the self-consistency threshold value is $\mu_0$-1.96 $\times$ $\sigma_0$, and to acquire the target gold-standard consistency of the doctors with different threshold value annotation and calculate a gold-standard consistency mean value $\mu_1$ and a gold-standard consistency variance $\sigma_1$, under the assumption that the target gold-standard consistency satisfies a Gaussian distribution, the gold-standard consistency threshold value is $\mu_1$-1.96 $\times$ $\sigma_1$. Thus, the self-consistency threshold value and the gold-standard consistency threshold value can be determined.

In addition, in the quality control method according to the first aspect of the present disclosure, optionally, the gathering is to compare each annotation result of each target fundus image in a plurality of groups of the target annotation results using an absolute majority voting method to determine the final annotation result of each target fundus image, and if the final annotation result is not able to be determined, the target fundus image is annotated as a difficult fundus image, and the difficult fundus image is annotated and arbitrated to obtain the final annotation result. Thus, the final annotation result can be obtained based on the absolute majority voting method.

In addition, in the quality control method according to the first aspect of the present disclosure, optionally, the gathering is to compare each annotation result of each target fundus image in a plurality of groups of the target annotation results, and in the case that each annotation result is consistent, taking the annotation result as the final annotation result of the target fundus image, while in the case that a plurality of annotation results are inconsistent, if the plurality of annotation results simultaneously include a same determination result and only one annotation result includes a determination result which is not identified in the other annotation results, the target fundus image is annotated as a fundus image to be quality-controlled, otherwise, the target fundus image is annotated as a difficult fundus image; quality control is performed on the fundus image to be quality-controlled and the final annotation result is obtained, and the difficult fundus image is annotated and arbitrated so as to obtain the final annotation result. In this case, the target fundus image can be divided into a target fundus image having a final annotation result, a fundus image to be quality-controlled, and a difficult fundus image and the final annotation result can be obtained by comparing each annotation result of each target fundus image among a plurality of groups of target annotation results.

In addition, in the quality control method according to the first aspect of the present disclosure, optionally, quality control is performed on the fundus image to be quality-controlled, and if it is determined that the unidentified determination result does not exist, the same determination result is taken as the final annotation result, while if it is determined that the unidentified determination result exists, the fundus image to be quality-controlled is taken as a difficult fundus image and the difficult fundus image is annotated and arbitrated to obtain the final annotation result. Thus, the fundus image to be quality-controlled can be divided into the fundus image to be quality-controlled having the final annotation result and the difficult fundus image, and the final annotation result can be obtained.

In addition, in the quality control method according to the first aspect of the present disclosure, optionally, the difficult fundus image is annotated and arbitrated by an arbitration doctor to obtain the final annotation result. Thus, the final annotation result of the difficult fundus image can be obtained.

In addition, in the quality control method according to the first aspect of the present disclosure, optionally, the standardization processing includes at least one of dividing the fundus images per the patient, unifying a name format of the fundus images, filtering out a non-fundus image, unifying a picture format of the fundus images, and unifying a background of the fundus images. Thus, the standardization processing on fundus images can be accomplished.

In addition, in the quality control method according to the first aspect of the present disclosure, optionally, the preliminary filtering includes dividing the standardized fundus images into at least two image quality grades including qualified and unqualified, the qualified fundus image is the standardized fundus image whose image quality grade is qualified. Thereby, the quality of the standardized fundus images can be preliminarily filtered to quickly obtain qualified fundus images.

In addition, in the quality control method according to the first aspect of the present disclosure, optionally, in the annotation, image quality of each image in the target fundus image set is classified into five image quality grades including very good, good, average, poor and very poor. In this case, the final annotation result can be subsequently determined in connection with a more detailed image quality grade.

In addition, in the quality control method according to the first aspect of the present disclosure, optionally, the disease includes at least one of diabetic retinopathy, hypertensive retinopathy, glaucoma, retinal vein occlusion, retinal artery occlusion, age-related macular degeneration, high myopia macular degeneration, retinal detachment, optic nerve disease, and congenital abnormalities of disc development. Thereby, at least one disease can be annotated.

In addition, in the quality control method according to the first aspect of the present disclosure, optionally, the preset condition is $d_{self} \leq D$ and $d_{gold} \leq D$, wherein $d_{self}$ is a self-evaluation index based on the self-consistency, $d_{gold}$ is a gold-standard evaluation index based on the gold-standard consistency, and $D$ is an evaluation index threshold value, the self-evaluation index $d_{self}$ satisfies the formula: $d_{self} = |J_{self} - \kappa_{self}|/\kappa_{self} \times 100\%$, wherein $J_{self} = SE_{self} + SP_{self} - 1$, $SE_{self}$ is sensitivity of the first annotation doctor obtained based on the two groups of annotation results for evaluating the self-consistency and $SP_{self}$ is specificity of the first annotation doctor obtained based on the two groups of annotation results for evaluating the self-consistency $\kappa_{self}$ is the self-consistency of the first annotation doctor, the gold-standard evaluation index $d_{gold}$ satisfies the formula: $d_{gold} = |J_{gold} - \kappa_{gold}|/\kappa_{gold} \times 100\%$, wherein $J_{gold} = SE_{gold} + SP_{gold} - 1$, $SE_{gold}$ is sensitivity of the first annotation doctor obtained on the basis of the two groups of annotation results for evaluating the gold-standard consistency, and $SP_{gold}$ is the specificity of the first annotation doctor obtained on the basis of the two groups of annotation results for evaluating the gold-standard consistency, and $\kappa_{gold}$ is the gold-standard consistency of the first annotation doctor. Thus, the preset condition can be determined based on the evaluation index threshold value.

A second aspect of the present disclosure provides a quality control system for data annotation on a fundus image, including: an acquisition module, used for acquiring a plurality of fundus images; a standardization processing module, used for performing standardization processing on each of the fundus images to obtain a plurality of standardized fundus images; a preliminary filtering module, used for performing preliminary filtering on the quality of each of the standardized fundus images to obtain a plurality of qualified fundus images; a data preparation module, used for preparing a target fundus image set, the target fundus image set includes a data set to be calibrated including the plurality of qualified fundus images, a gold-standard data set including a first preset number of gold-standard fundus images with a known correct annotation result, and a self-consistency determination data set composed of at least one image in the data set to be calibrated, each image of the target fundus image set is taken as each target fundus image; a annotation module, used for acquiring a plurality of groups of doctor annotation results by a plurality of first annotation doctors respectively annotating each image in the target fundus image set, the doctor annotation results include at least one determination result, the determination result at least includes disease information of no obvious abnormality or of a disease; an evaluation module, used for calculating a self-consistency and a gold-standard consistency of a corresponding first annotation doctor based on the doctor annotation result so as to obtain the doctor annotation result of the first annotation doctor satisfying a preset condition as a target annotation result, the self-consistency is obtained by taking any one of two groups of annotation results of the doctor annotation result of each image in the self-consistency determination data set and the doctor annotation result of an image, which is repeated with respective image in the self-consistency determination data set, in the data set to be calibrated as a first group of annotation results and the other group as a second group of annotation results and performing evaluation using a self-consistency determination and evaluation method, the gold-standard consistency is obtained by taking the correct annotation result of the gold-standard data set as a first group of annotation results and the doctor annotation result of each image in the gold-standard data set as a second group of annotation results and using a gold-standard consistency determination and evaluation method; a gathering module, used for gathering the plurality of groups of the target annotation results to obtain a final annotation result. In this case, on the basis of the gold-standard data set and the self-consistency determination data set, the doctor annotation result of the first annotation doctor that satisfies the preset conditions can be obtained as the target annotation result and gathered. Thus, the accuracy of data annotation of the fundus image can be improved.

According to the present disclosure, it is able to provide a quality control method and a quality control system for data annotation on fundus images with high accuracy.

## BRIEF DESCRIPTION OF THE DRAWINGS

The present disclosure will now be explained in further detail, by way of example only, with reference to the accompanying drawings.

Fig. 1 is a use scenario diagram illustrating a quality control method for data annotation on a fundus image according to an example of the present disclosure.

Fig. 2 is a flowchart illustrating a quality control method for data annotation on a fundus image according to an example of the present disclosure.

Fig. 3 is a block diagram illustrating a target fundus image set according to an example of the present disclosure.

Fig. 4 is a flowchart illustrating the determination of a self-consistency threshold value according to an example of the present disclosure.

Fig. 5 is a statistical chart illustrating target self-consistency and target gold-standard consistency according to an example of the present disclosure.

Fig. 6 is a flowchart illustrating the manner in which the examples of the present disclosure are gathered.

Fig. 7 is a flowchart illustrating quality control of a fundus image to be quality-controlled and obtaining a final annotation result according to an example of the present disclosure.

Fig. 8 is a block diagram showing a quality control system for data annotation on a fundus image according to an example of the present disclosure.

## DETAILED DESCRIPTION

Hereinafter, preferred embodiments of the present disclosure will be described in detail with reference to the accompanying drawings. In the following description, the same components are given the same reference characters, and repeated description is omitted. In addition, the drawings are merely schematic, and the proportions of the dimensions of the parts relative to each other, or the shapes of the parts, etc. may differ from the reality. It should be noted that the terms "comprising" and "having", and any variations thereof, in this disclosure, such as a process, method, system, product, or device that comprises or has a list of steps or elements are not necessarily limited to those explicitly recited steps or elements, but may include or have other steps or elements not explicitly recited or inherent to such process, method, product, or device. All methods described in this disclosure can be performed in any suitable order unless otherwise indicated herein or otherwise clearly contradicted by context.

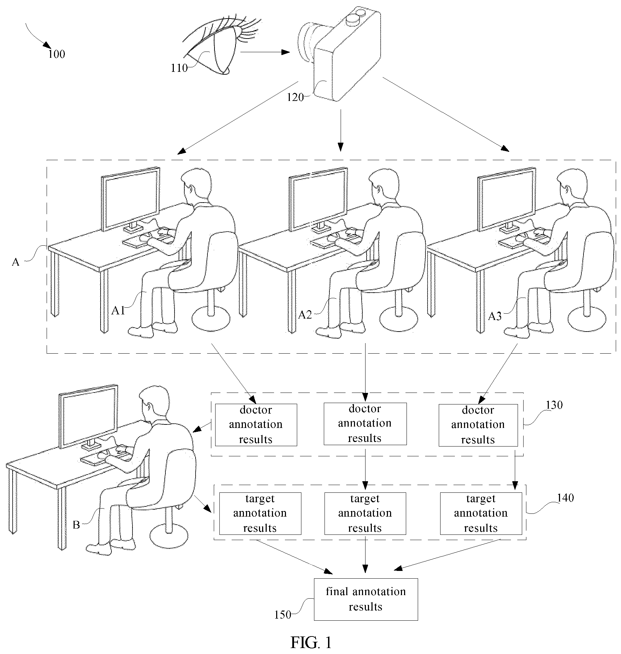

Fig. 1 is a use scenario diagram illustrating a quality control method for data annotation on a fundus image according to an example of the present disclosure. In some examples, a quality control method for data annotation on a fundus image (sometimes also referred to simply as a quality control method) according to the present disclosure may be applied to a use scenario 100 as shown in Fig. 1. In the use scenario 100, first, there may be a plurality of first annotation doctors A, for example, three first annotation doctors A, first annotation doctor A1, first annotation doctor A2, and first annotation doctor A3, that annotate a plurality of fundus images of the fundus of a plurality of human eyes 110 to obtain a doctor annotation result 130 (described later). Next, a doctor annotation result 130 meeting a preset condition (described later) may be taken as a target annotation result 140 (described later). For example, as shown in Fig. 1, assuming that the doctor annotation results 130 of the first annotation doctor A2 and the first annotation doctor A3 meet the self-consistency and gold-standard consistency requirements, the doctor annotation results

130 of the first annotation doctor A2 and the first annotation doctor A3 may be taken as the target annotation results 140. Finally, the target annotation results 140 may be gathered to obtain a final annotation result 150 (described later).

In some examples, the fundus of the human eye 110 refers to tissue in the posterior portion of the eyeball, which may include the inner membrane, retina, macula, and blood vessels of the eyeball. In some examples, a fundus image of a fundus of human eyes 110 may be obtained by the acquisition device 120. In some examples, the acquisition device 120 may include, but is not limited to, a camera or the like. The camera may be, for example, a color fundus camera.

In some examples, a doctor annotation result 130 that does not meet the preset condition may be re-annotated by a second annotation doctor B and ultimately a target annotation result 140 is obtained. However, the examples of the present disclosure are not limited hereto, in other examples, doctor annotation results 130 that do not meet preset conditions may be filtered out. In some examples, the second annotation doctor (s) B may be one or more. In some examples, if the doctor annotation result 130 re-annotated by one second annotation doctor B does not meet the preset condition, the re-annotation may be continued by another second annotation doctor B until the doctor annotation result 130 meeting the preset condition is obtained as the target annotation result 140. In some examples, the second annotation doctor B may be different from the first annotation doctor A. In some examples, the second annotation doctor B and the first annotation doctor A may include, but are not limited to, a professional ophthalmologist or an experienced doctor.

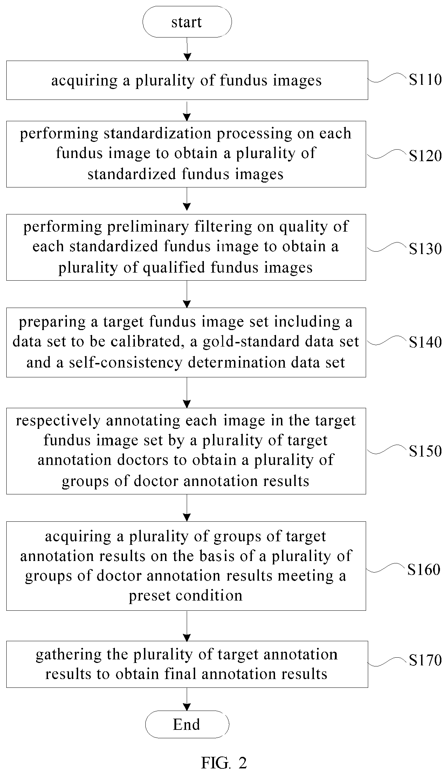

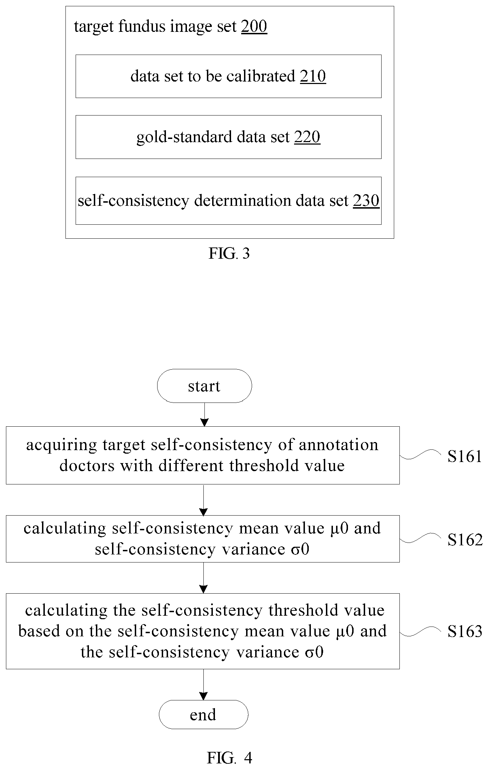

Hereinafter, a quality control method according to the present disclosure will be described in detail with reference to the accompanying drawings. Fig. 2 is a flowchart illustrating the quality control method for data annotation on a fundus image according to an example of the present disclosure. According to the invention as defined in claim 1 and as shown in figure 2, the quality control method includes acquiring a plurality of fundus images (step S110), performing standardization processing on each fundus image to obtain a plurality of standardized fundus images (step S120), performing preliminary filtering on quality of each standardized fundus image to obtain a plurality of qualified fundus images (step S130), preparing a target fundus image set including a data set to be calibrated (see Fig. 3), a gold-standard data set and a self-consistency determination data set (step S140), annotating each image in the target fundus image set by a plurality of first annotation doctors respectively to obtain a plurality of groups of doctor annotation results (step S150), acquiring a plurality of groups of target annotation results based on the plurality of groups of doctor annotation results meeting a preset condition (step S160), and gathering the plurality of groups of target annotation results to obtain a final annotation result (step S170). In this case, on the basis of the gold-standard data set and the self-consistency determination data set, the doctor annotation result of the first annotation doctor that satisfies the preset conditions can be obtained as the target annotation result and gathered. Thus, the accuracy of data annotation of the fundus image can be improved.

According to the invention as defined in claim 1, in step S110, a plurality of fundus images is acquired. In some examples, the fundus image may be a color fundus image. The color fundus images can clearly show the rich fundus information such as optic disc, optic cup, macula blood vessels,etc. In addition, the fundus image may be a RGB mode or a grayscale mode image or the like. In some examples, the fundus image may be a fundus image acquired by the acquisition device 120. In other examples, the fundus image may be an image pre-stored in the server. In some examples, the plurality of fundus images may be, for example, 5-200,000 fundus images from a cooperative hospital with patient information removed.

According to the invention as defined in claim 1, in step S120, standardization processing is performed on each fundus image to obtain a plurality of standardized fundus images. In some examples, the standardization processing may include at least one of classifying the fundus image per the patient, unifying a name format of the fundus images, filtering out a non-fundus image, unifying a picture format of the fundus images, and unifying a background of the fundus images. Additionally, in some examples, the non-fundus image may include, but is not limited to, a fundus mosaic or an anterior segment map. In some examples, the non-fundus image may be an image other than a 45-degree fundus image centered on the optic disc and macula. In addition, in some examples, the name format of the fundus image may be unified, e.g., the patient information in the name of the fundus image may be removed and the name of the fundus image may be standardized. Additionally, in some examples, the name of the fundus image may be turned into a hash value. In addition, in some examples, the picture format of the fundus images (e.g., jpg format) may be unified. Additionally, in some examples, the background of the fundus images may be unified (e.g., the fundus images may be unified into a black background).

According to the invention as defined in claim 1, in step S130, the quality of each of the standardized fundus images is preliminarily filtered to obtain a plurality of qualified fundus images.

In some examples, the preliminary filtering may include classifying the standardized fundus images into at least two image quality grades including qualified and unqualified. Thereby, the quality of the standardized fundus images can be preliminarily filtered to quickly acquire qualified fundus images.

In some examples, the quality of the standardized fundus image may be determined by a plurality of first annotation doctors A to classify the standardized fundus image into a plurality of image quality grades. In some examples, the standardized fundus image may be ranked based on factors that affect the quality of the fundus image. In some examples, factors affecting the quality of the fundus image may include, but are not limited to, at least one of a location at which the fundus image was taken, an exposure of the fundus image, and a definition of the fundus image. For example, a standardized fundus image with an acceptable image quality grade may be an image with the correct location, moderate exposure, and good definition. In this case, the quality of the standardized fundus image is ranked. Thus, it is facilitated to obtain a qualified fundus image.

However, the examples of the present disclosure are not limited hereto, in other examples, the standardized fundus images may be classified in more precise way in the preliminary filtering. For example, the standardized fundus image may be classified into at least five image quality grades. In some examples, the five image quality grades may include very good, good, average, poor, and very poor. In some examples, the image quality grade may also include unreadable images caused by abnormalities in the shot region (e.g., non-fundus images), no image or image acquisition technique issues, and other issues. In some examples, the image quality grades may be qualified, barely qualified, and unqualified.

Additionally, according to the invention as defined in claim 1, in step S130, a plurality of qualified fundus images is acquired. In some examples, the qualified fundus image may be a standardized fundus image with a qualified image quality grade. However, the examples of the present disclosure are not limited thereto, in other examples, a qualified fundus image may be a standardized fundus image with image quality grades of very good, good, average, and poor. The qualified fundus image may be a standardized fundus image of which image quality grade is very good, good and average or the qualified fundus image may be a standardized fundus image of which image quality grade is very good and good. In other examples, a qualified fundus image may be a standardized fundus image with an image quality grade of qualified and barely qualified. Thus, a qualified fundus image can be obtained.

Fig. 3 is a block diagram illustrating a target fundus image set according to the invention as defined in claim 1. As described above, the quality control method includes step S140 (see Fig. 2). According to the invention as defined in claim 1, in step S140, a target fundus image set 200 including a data set to be calibrated 210, a gold-standard data set 220, and a self-consistency determination data set 230 is prepared. As shown in Fig. 3, according to the invention as defined in claim 1, the target fundus image set 200 includes a data set to be calibrated 210, a gold-standard data set 220, and a self-consistency determination data set 230.

According to the invention as defined in claim 1, the data set to be calibrated 210 includes a plurality of qualified fundus images. In some examples, the data set to be calibrated 210 may include all qualified fundus images obtained in step S130. In some examples, the data set to be calibrated 210 may include the partial qualified fundus images obtained in step S130. In some examples, all qualified fundus images obtained in step S130 may be grouped and each group of qualified fundus images may be taken as one data set to be calibrated 210. For example, all of the qualified fundus images obtained in step S130 may be grouped in a group of 80, 90 or 100 images.

Additionally, according to the invention as defined in claim 1, gold-standard data set 220 includes a first preset number of gold-standard fundus images. The gold-standard fundus image may be a fundus image for which correct annotation results are known. In some examples, the gold-standard fundus image may be a fundus image of a known correct annotation result from an annotation database. In some examples, the first preset number may be 5 to 20. For example, the first preset number may be 5, 10, 15, or 20, etc. However, the examples of the present disclosure are not limited thereto, and in other examples, the first preset number may be other values.

Additionally, according to the invention as defined in claim 1, the self-consistency determination data set 230 consists of images in the data set to be calibrated 210. In some examples, the number of images in the self-consistency determination data set 230 may be at least one. In some examples, the number of images in the self-consistency determination data set 230 may be 5 to 20. For example, the number of images in the self-consistency determination data set 230 may be 5, 10, 15, or 20, etc. However, the examples of the present disclosure are not limited hereto, in other examples, the number of images in the self-consistency determination data set 230 may be other values. In some examples, the number of images in the self-consistency determination data set 230 may be less than the number of images in the data set to be calibrated 210. Thus, the image in the self-consistency determination data set 230 can be repeated with part of the images in the data set to be calibrated 210. Additionally, in some examples, each image of the target fundus image set 200 may serve as each target fundus image.

According to the invention as defined in claim 1, in step S150, each image in the target fundus image set 200 is annotated by a plurality of first annotation doctors A to obtain a plurality of groups of doctor annotation results 130. For example, assuming that three first annotation doctors A annotate the target fundus image set 200 respectively, three first annotation doctors A may obtain three groups of doctor annotation results 130. In some examples, a plurality of first annotation doctors A may annotate each image in the target fundus image set 200 using an online annotation system. In some examples, the number of first annotation doctors A may be greater than or equal to three. For example, the number of first annotation doctors A may be 3, 5, 7, or 9, etc.

According to the invention as defined in claim 1, the doctor annotation results 130 for each image in the target fundus image set 200 include at least one determination result. According to the invention as defined in claim 1, the determination result includes disease information of no obvious abnormality or of a disease. In some examples, if there is not any disease in the image of the target fundus image set 200, the doctor annotation result 130 for that image may be no obvious abnormality. In some examples, the doctor annotation result 130 may be a determination result of multiple diseases. For example, the doctor annotation result 130 may be diabetic retinopathy stage I and the presence of glaucoma.

In some examples, the doctor annotation result 130 may include eye difference (e.g., left or right eye) and an image quality grade of the quality of each image in the target fundus image set 200. In some examples, if the quality of each standardized fundus image is not classified in more precise way in the preliminary filtering, the first annotation doctor A may classify the quality of each image in the target fundus image set 200 in more precise way in the annotation process. In other examples, if the quality of each standardized fundus image is classified in more precise way in the preliminary filtering, the first annotation doctor A may re-classify the quality of each image in the target fundus image set 200 in the annotation process. Specific contents are described with reference to a more detailed classification of the standardized fundus image. In this case, the final annotation result 150 may be subsequently determined in conjunction with a more detailed image quality grade. In some examples, the image quality grades in the doctor annotation result 130 may include image quality grades obtained by the preliminary filtering and image quality grades obtained in the annotation process.

In some examples, the disease can include at least one of diabetic retinopathy, hypertensive retinopathy, glaucoma, retinal vein occlusion, retinal artery occlusion, age-related macular degeneration, high myopia macular degeneration, retinal detachment, optic nerve disease, congenital abnormalities of disc development. Thereby, at least one disease can be annotated. However, the examples of the present disclosure are not limited thereto, and the quality control method of the present disclosure can be easily generalized to quality control of data annotation of other diseases or data annotation of other fields. In some examples, the disease information may be a staging based on the severity of the disease. For example, diabetic retinopathy can be staged as stage I, II, III, IV, V, and VI. In other examples, the disease information may be the presence of a certain disease, e.g., the disease information may be the presence of glaucoma.

As described above, the quality control method may include step S160 (see Fig. 2). In some examples, in step S160, a plurality of groups of target annotation results 140 may be obtained on the basis of a plurality of groups of doctor annotation results 130 meeting a preset condition. In some examples, in step S160, the self-consistency and gold-standard consistency of the corresponding first annotation doctor A may be calculated based on the doctor annotation result 130.

As described above, each image of the target fundus image set 200 may serve as each target fundus image. In some examples, self-consistency may be obtained by determining whether the doctor annotation results 130 obtained by each first annotation doctor A annotating the same target fundus image twice are consistent or not. In some examples, it can be illustrated that the higher the self-consistency, the more stable the annotation level of the first annotation doctor A may be. Specifically, in some examples, in calculating self-consistency, the doctor annotation results 130 of each image in the self-consistency determination data set 230 can be obtained, as well as the doctor annotation results 130 of images in the data set to be calibrated 210 that are repeated with respective image in the self-consistency determination data set 230. In some examples, it is to take any one of the two groups of annotation results as a first group of annotation results and the other set as a second group of annotation results and evaluate using a self-consistency determination and evaluation method to obtain self-consistency.

In some examples, the self-consistency determination method may use a quadratic weighted kappa coefficient to calculate the disease self-consistency of each of the first annotation doctors A for each of the diseases. In some examples, the quadratic weighted kappa coefficient $\kappa$ for a single disease may be

$$\kappa = 1 - \frac{\sum_{i,j} W_{ij} X_{ij}}{\sum_{i,j} W_{ij} E_{ij}}$$

wherein $W_{ij}$ may represent a quadratic weighting coefficient, $X_{ij}$ may represent a number of target fundus images in which the determination result in the first group of annotation results is $i$ and the determination result in the second group of annotation results is $j$, $E_{ij}$ may represent an expected number of target fundus images in which the determination result in the first group of annotation results is i and the determination result in the second group of annotation results is $j$ may be represented. In some examples, when i is not equal to $j$, $E_{ij}$ may be zero. In some examples, a quadratic weighting factor $W_{ij}$ may be set as needed to highlight the importance of a certain determination result. Thus, it is able to check consistency between the first group of annotation results and the second group of annotation results.

In some examples, in the self-consistency determination method, self-consistency of each disease may be weighted to calculate the self-consistency of each first annotation doctor A. For example, the weight for disease self-consistency of diabetic retinopathy can be set to 1, and the weight for disease self-consistency of other diseases can be set to 0.5. Thus, the self-consistency of each first annotation doctor A can be calculated based on the self-consistency determination method. However, the examples of the present disclosure are not limited hereto, and in other examples, self-consistency may be calculated in other ways.

According to the invention as defined in claim 1, in step S160, the gold-standard consistency of the corresponding first annotation doctor A is calculated on the basis of the doctor annotation result 130. Specifically, according to the invention as defined in claim 1, in calculating the gold-standard consistency, the correct annotation result for the gold-standard data set 220 is taken as the first group of annotation results, that is, the gold-standard fundus image is taken as the first group of annotation results, and the doctor annotation results 130 for each image in the gold-standard data set 220 is taken as the second set of annotation results. According to the invention as defined in claim 1, the gold-standard consistency is obtained on the basis of the first group of annotation results and the second group of annotation results and evaluated using a gold-standard consistency determination and evaluation method.

In some examples, the gold-standard consistency determination method may use a quadratic weighted kappa coefficient to calculate the disease gold-standard consistency for each first annotation doctor A to determine each disease. In some examples, gold-standard consistency of each disease may be weighted to calculate a gold-standard consistency for each first annotation doctor A. Thus, the self-consistency of each first annotation doctor A can be calculated on the basis of the gold-standard consistency determination method. The detailed description of the self-consistency determination method can be referenced for specific contents. However, the examples of the present disclosure are not limited hereto, and in other examples, the gold-standard consistency may be calculated in other ways.

In some examples, in step S160, the doctor annotation result 130 of the first annotation doctor A meeting the preset condition may be obtained and the doctor annotation result 130 may be taken as the target annotation result 140. In some examples, the preset condition may be $d_{self} \leq D$ and $d_{gold} \leq D$, wherein, $d_{self}$ is a self-evaluation index based on self-consistency, $d_{gold}$ is a gold-standard evaluation index based on gold-standard consistency, and $D$ is an evaluation index threshold value. In some examples, $D \leq 5$ %. Thus, the preset condition can be determined based on the evaluation index threshold value.

In some examples, the self-evaluation index $d_{self}$ may satisfy the formula: $d_{self} = |J_{self} - \kappa_{self}|/\kappa_{self} \times 100\%$, wherein $J_{self} = SE_{self} + SP_{self} - 1$, $SE_{self}$ is a sensitivity of the first annotation doctor A obtained on the basis of the two groups of annotation results for evaluating self-consistency, $SP_{self}$ is the specificity of the first annotation doctor A obtained on the basis of the two groups of annotation results for evaluating self-consistency, $\kappa_{self}$ is self-consistency of the first annotation doctor A. In some examples, any group of the two groups of annotation results used to evaluate self-consistency may be taken as a gold-standard to evaluate the other group to obtain the sensitivity and specificity of the first annotation doctor A.

In some examples, the gold-standard evaluation index $d_{gold}$ may satisfy the formula: $d_{gold} = |J_{gold} - \kappa_{gold}|/\kappa_{gold} \times 100\%$, wherein $J_{gold} = SE_{gold} + SP_{gold} - 1$, $SE_{gold}$ is a sensitivity of the first annotation doctor A obtained on the basis of the two groups of annotation results for evaluating the gold-standard consistency, and $SP_{gold}$ is a specificity of the first annotation doctor A obtained on the basis of the two groups of annotation results for evaluating the gold-standard consistency, $\kappa_{gold}$ is the gold-standard consistency of the first annotation doctor A. In some examples, the first group of the two groups of annotation results used to evaluate gold-standard consistency may be taken as the gold-standard to evaluate the second group of annotation results to obtain the sensitivity and specificity of the first annotation doctor A.

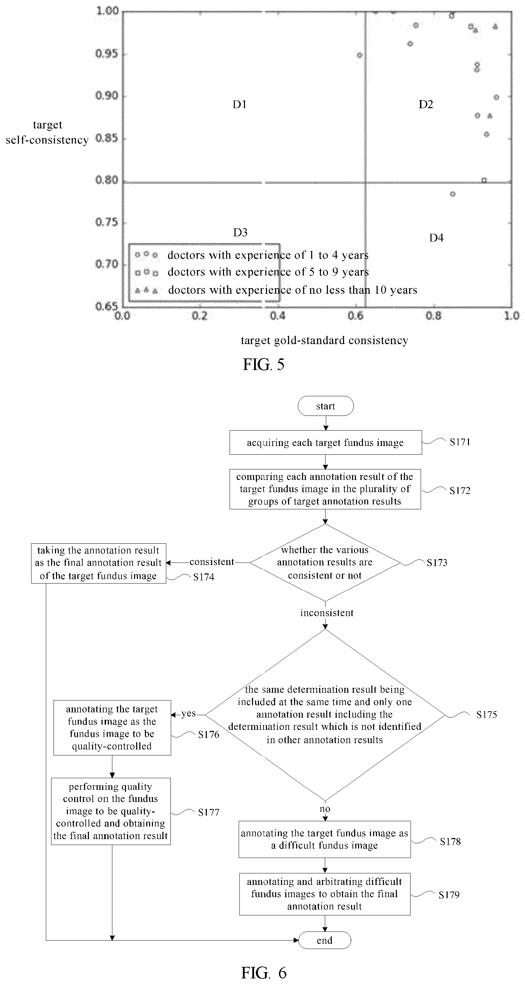

Fig. 4 is a flowchart illustrating the determination of a self-consistency threshold value according to an example of the present disclosure. Fig. 5 is a statistical chart illustrating target self-consistency and target gold-standard consistency according to an example of the present disclosure. The first region D1, the second region D2, the third region D3, and the fourth region D4 are four regions in the statistical chart. In some examples, the preset condition may be that the self-consistency is greater than the self-consistency threshold value and the gold-standard consistency is greater than the gold-standard consistency threshold value. In some examples, it may be to analyze the target self-consistency and target gold-standard consistency of doctors with different threshold value annotation and use abnormality detection to determine the self-consistency threshold value and gold-standard consistency threshold value. Thus, the self-consistency threshold value and the gold-standard consistency threshold value can be determined.

In some examples, as shown in Fig. 4, the process of determining a self-consistency threshold value based on an abnormality detection approach may include obtaining target self-consistency for annotation doctors with different threshold value (step S161), calculating a self-consistency mean value $\mu_0$ and a self-consistency variance $\sigma_0$ (step S162), and calculating a self-consistency threshold value based on the self-consistency mean $\mu_0$ and the self-consistency variance $\sigma_0$ (step S163). Thus, the self-consistency threshold value can be determined.

In some examples, in step S161, target self-consistency for annotation doctors with different threshold values may be obtained. Specifically, the target self-consistency of annotation doctors with different threshold values may be analyzed. For example, the target self-consistency of a threshold value annotation doctor with an experience of 1-4 years, of a threshold value annotation doctor with an experience of 5-9 years, and of a threshold value annotation doctor with an experience of no less than 10 years may be analyzed. In some examples, the target self-consistency and the target gold-standard consistency (described later) may be obtained simultaneously. As an example of the target self-consistency and target gold-standard consistency statistics, Fig. 5 illustrates the statistic results of target self-consistency and target gold-standard consistency for threshold value annotation doctors with different seniority. Wherein the circle may represent the target self-consistency and target gold-standard consistency of the threshold value annotation doctor with an experience of 1-4 years. The square may represent the target self-consistency and target gold-standard consistency of the threshold value annotation doctor with an experience of 5-9 years. The triangle may represent the target self-consistency and target gold-standard consistency of the threshold value annotation doctor with an experience of no less than ten years. The first region D1, the second region D2, the third region D3, and the fourth region D4 are four regions in the statistical chart. It can be seen from Fig. 5 that the statistical results of the target self-consistency and the target gold-standard consistency of threshold value annotation doctors with an experience of 1-4 years fall into the first region D1 and the fourth region D4, while the statistical results of the target self-consistency and the target gold-standard consistency of the threshold value annotation doctors with an experience of no less than 5 years mainly fall into the second region D2.

In some examples, in step S162, a self-consistency mean value $\mu_0$ and a self-consistency variance $\sigma_0$ may be calculated. In some examples, the self-consistency mean value $\mu_0$ and the self-consistency variance $\sigma_0$ of the target self-consistency may be calculated.

In some examples, in step S163, a self-consistency threshold value may be calculated based on the self-consistency mean value $\mu_0$ and the self-consistency variance $\sigma_0$. Specifically, in some examples, the self-consistency threshold value may be $\mu_0$-1.96×$\sigma_0$ under the assumption that the target self-consistency satisfies a Gaussian distribution. In this case, the probability of an anomaly occurring is less than 2.5%. In some examples, the self-consistency threshold value may be 0.7977.

In some examples, the process of determining the gold-standard consistency threshold value based on the manner of abnormality detection may include obtaining target gold-standard consistency of annotation doctors with different threshold values, calculating a gold-standard consistency mean value $\mu_1$ and a gold-standard consistency variance $\sigma_1$ of the target gold-standard consistency, and calculating the gold-standard consistency threshold value based on the gold-standard consistency mean value $\mu_1$ and the gold-standard consistency variance $\sigma_1$. Thus, the gold-standard consistency threshold value can be determined. In some examples, the gold-standard uniformity threshold value may be $\mu_1$-1.96×$\sigma_1$ under the assumption that the target gold-standard uniformity satisfies a Gaussian distribution. In this case, the probability of an anomaly occurring is less than 2.5%. In some examples, the gold-standard consistency threshold value may be 0.6235. A detailed description of the process for determining the gold-standard consistency threshold value can be found in the process for determining the self-consistency threshold value for reference and will not be described in detail herein.

However, the examples of the present disclosure are not limited hereto, in other examples, abnormality detection in other way may be used to determine the self-consistency threshold value and the gold-standard consistency threshold value.

In some examples, in step S160, the doctor annotation result 130 of the first annotation doctor A which does not meet the preset condition may be re-annotated by the second annotation doctor B for each image in the target fundus image set 200. In some examples, the doctor annotation result 130 that does not meet the preset condition may be continually re-annotated until the doctor annotation result 130 that meets the preset condition is obtained as the target annotation result 140. In this case, the doctor annotation result 130 of the first annotation doctor A which does not meet the preset condition is re-annotated. Thus, the target annotation result 140 can be obtained. In some examples, the second annotation doctor may be different from the first annotation doctor in step S150.

Fig. 6 is a flowchart illustrating the manner in which the examples of the present disclosure are gathered. According to the invention as defined in claim 1, the quality control method includes step S170 (see Fig. 2). In step S170, a plurality groups of target annotation results 140 is gathered to obtain a final annotation result 150.

In some examples, absolute majority voting is used to compare each annotation result of each target fundus image in the plurality of groups of target annotation results 140 to determine a final annotation result 150 for each target fundus image. Specifically, when individual annotation results are compared to each other using Absolute Majority Voting, the annotation results will be accepted as part of the final annotation result 150 if more than half of determination results of the annotation results are consistent (that is, more than half of the valid votes are required to be accepted). In some examples, if the final annotation result 150 cannot be determined (i.e., the number of valid votes is not more than half), the target fundus image is annotated as a difficult fundus image. In some examples, difficult fundus images may be annotated and arbitrated to obtain a final annotation result 150. Thus, the final annotation result 150 can be obtained based on the absolute majority voting method.

In some examples, difficult fundus images may be annotated by an arbitration doctor to obtain an arbitration annotation result. In some examples, the arbitration annotation result may include at least one determination result. In some examples, the arbitration annotation result may be taken as the final annotation result 150.

However, the examples of the present disclosure are not limited thereto, in other examples, as shown in Fig. 6, the process of the gathered manner of step S170 may include steps S171 to S179. In this case, by comparing the respective annotation results of the respective target fundus images in the plurality of groups of target annotation results 140, the target fundus image can be divided into a target fundus image having a final annotation result 150, a fundus image to be quality-controlled, and a difficult fundus image, and the final annotation result 150 can be obtained.

In some examples, in step S171, each target fundus image may be acquired. Specifically, in some examples, each target fundus image in the target fundus image set 200 may be traversed sequentially and compared in step S172.

In some examples, in step S172, the respective annotation results of the respective target fundus images in the plurality of sets of target annotation results 140 obtained in step S171 may be compared. For example, assuming there are three groups of target annotation results 140, each target fundus image may have three annotation results originated from each group of target annotation results 140.

In some examples, in step S173, it may be determined whether the respective annotation results are consistent. For example, it is able to compare the three annotation results of step S172 and make sure whether they are identical or not.

In some examples, if the respective annotation results are consistent, the process may proceed to step S174. In some examples, in step S174, the annotation result may be taken as the final annotation result 150 of the target fundus image. In some examples, it may be determined that the respective annotation results are consistent when the determination results included in the respective annotation results aretotally identical. For example, if there is no obvious abnormality in each annotation result, it can be determined that each annotation result is consistent. For another example, if each annotation result is stage I diabetic retinopathy and glaucoma is present, it can be determined that each annotation result is consistent.

In some examples, if the plurality of annotation results are inconsistent, step S175 may be entered. In some examples, in step S175, it may be determined whether each annotation result includes the same determination result at the same time and only one annotation result includes a determination result which is not identified in other annotation results, in case of"yes", step S176 may be entered, otherwise, step S177 may be entered.

For example, assume that the plurality of annotation results of the target fundus image are a first annotation result, a second annotation result, and a third annotation result, respectively, wherein the first annotation result is diabetic retinopathy stage I, the second annotation result is diabetic retinopathy stage I, and the third annotation result is diabetic retinopathy stage I and presence of glaucoma. In this case, diabetic retinopathy stage I is the same determination result included in each annotation result at the same time. Presence of glaucoma is an unidentified determination result, and only one annotation result includes presence of glaucoma. However, the examplesof the present disclosure are not limited to hereto, and in other examples, the determination may be made by other determination conditions. For example, the condition that each annotation result may include the same determination result at the same time, and at least one annotation result may include a determination result that is not recognized in the other annotation results is taken as the determination condition in step S175.

In some examples, in step S176, the target fundus image may be annotated as a fundus image to be quality controlled.

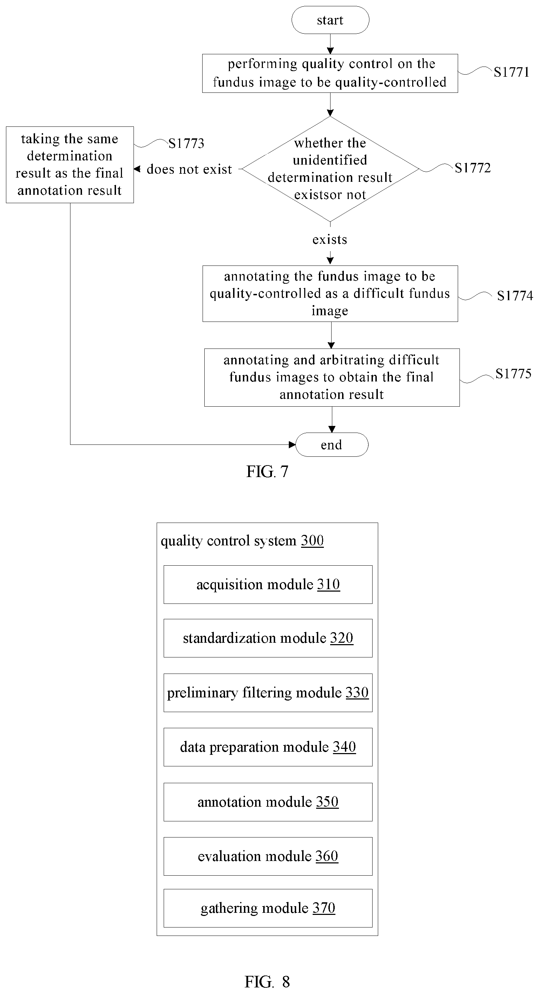

Fig. 7 is a flowchart illustrating quality control of a fundus image to be quality-controlled and obtaining a final annotation result according to an example of the present disclosure. In some examples, in step S177, the fundus image to be quality-controlled may be quality-controlled and a final annotation result 150 is obtained. As shown in Fig. 7, in some examples, the process of qualitycontrolling the fundus image to be quality-controlled and obtaining the final annotation result may include steps S1771 to S1775.

In some examples, in step S1771, quality control may be performed on the fundus image to be quality-controlled. In some examples, thefundus image to be quality-controlled may be quality-controlled by a quality control doctor to obtain a quality control determination result. In some examples, the unidentified determination result in the fundus image to be quality-controlled (for example, only one annotation result described in step S175 includes the presence of glaucoma) may be evaluated to obtain the quality control determination result (for example, there are unrecognized determination results or there are no unidentified determination results).

In some examples, in step S1772, it may be identified whether unidentified determination result exists or not on the basis of the quality control determination result of step S 1771, in case of "no", step S1773 may be entered, otherwise, step S1774 may be entered.

In some examples, in step S 1773, the same determination result is taken as the final annotation result 150 in step S 1773. For example, assuming that the plurality of annotation results of the target fundus image are a first annotation result, a second annotation result, and a third annotation result, respectively, wherein the first annotation result is diabetic retinopathy stage I, the second annotation result is diabetic retinopathy stage I, the third annotation result is diabetic retinopathy stage I with presence of glaucoma. In this case, the diabetic retinopathy stage I is the same determination result that each annotation result simultaneously includes, and can be taken as the final annotation result 150 of the target fundus image.

In some examples, in step S1774, the fundus image to be quality-controlled may be annotated as a difficult fundus image.

In some examples, in step S1775, difficult fundus images may be annotated and arbitrated to obtain a final annotation result 150. In some examples, difficult fundus images may be annotated by an arbitration doctor to obtain an arbitration annotation result. In some examples, the arbitration annotation result may include at least one determination result. In some examples, the arbitration annotation result may be taken as the final annotation result 150. In some examples, a final annotation result 150 may be obtained on the basis of a plurality of target annotation results 140, quality control determination results, and arbitration annotation results for a difficult fundus image.

As described above, the process of the gathering of step S170 may include step S178. In some examples, in step S178, the target fundus image may be marked as a difficult fundus image.

In some examples, in step S179, difficult fundus images may be annotated and arbitrated to obtain a final annotation knot 150. In some examples, a final annotation result 150 may be obtained on the basis of a plurality of target annotation results 140 and arbitration annotation results for a difficult fundus image. Thus, the final annotation result 150 of the difficult fundus image can be obtained. The detailed content can be seen in relevant description in step S1775.

In some examples, the doctor annotation results 130 of the target fundus image set 200 may be counted to obtain statistical results. In some examples, the statistical results may include a gold-standard consistency re-annotation ratio. In some examples, the gold-standard consistency re-annotation ratio may be the ratio of the re-annotated target fundus image in the target fundus image set 200 due to the unqualified gold-standard consistency. In some examples, the statistical results may include a self-consistency re-annotation ratio. In some examples, self-consistency re-annotation ratio may be the ratio of the re-annotated target fundus images in the target fundus image set 200 due to unqualified self-consistency.

In some examples, the annotation process may be quality-controlled on the basis of statistical results. For example, if the gold-standard consistency re-annotation ratio exceeds a pre-set value, the assignment of annotation tasks to relevant annotation doctors may be subsequently reduced or cancelled.

In some examples, annotation reports may be output. In some examples, the annotation report may include at least one of a doctor annotation result 130, a target annotation result 140, a final annotation result 150, a quality control determination result, an arbitration annotation result, and a statistical result.

Hereinafter, the quality control system 300 for data annotation on a fundus image according to the present disclosure will be described in detail with reference to Fig. 8. The quality control system 300 for data annotation on a fundus image in the present disclosure may sometimes be referred to simply as "quality control system 300". The quality control system 300 is used to implement the quality control method described above. Fig. 8 is a block diagram illustrating a quality control system for data annotation on a fundus image according to the invention as defined in claim 15.

According to the invention as defined in claim 15, as shown in Fig. 8, the quality control system 300 includes an acquisition module 310, a standardization processing module 320, a preliminary filtering module 330, a data preparation module 340, an annotation module 350, an evaluation module 360, and a gathering module 370. The acquisition module 310 is configured to acquire a plurality of fundus images. The standardization processing module 320 is configured to perform standardization processing on each of the plurality of fundus image to obtain a plurality of standardized fundus images. The preliminary filtering module 330 is configured to perform preliminary filtering on quality of each of the standardized fundus images to obtain a plurality of qualified fundus images. The data preparation module 340 is configured to prepare the target fundus image set 200 including the data set to be calibrated 210, the gold-standard data set 220, and the self-consistency determination data set 230. The annotation module 350 is configured to obtain a plurality of groups of doctor annotation results 130 for each image in the target fundus image set by a plurality of first annotation doctors A, respectively. The evaluation module is configured to obtain a plurality of sets of target annotation results 140 meeting preset condition on the basis of a plurality of groups of doctor annotation results. The gathering module 370 is configured to gather a plurality of groups of target annotation results 140 to obtain a final annotation result 150. In this case, the doctor annotation result 130 of the first annotation doctor A meeting the preset condition may be acquired as the target annotation result 140 and gathered on the basis of the gold-standard data set and the self-consistency determination data set. Thus, the accuracy of data annotation of the fundus image can be improved.

In some examples, in the acquisition module 310, the fundus image may be a color fundus image. The color fundus images can clearly show the rich fundus information such as optic disc, optic cup, macula blood vessels. The detailed description may refer to the relevant description of step S110, which will not be repeated here.

In some examples, in the standardization processing module 320, the standardization processing may include at least one of classifying the fundus images per patient, unifying a name format of the fundus image, filtering out a non-fundus image, unifying a picture format of the fundus image, and unifying a background of the fundus image. Thus, the standardization processing on fundus images can be performed. The detailed description may refer to the relevant description of step S120, which will not be repeated here.

In some examples, in the preliminary filtering module 330, the preliminary filtering may classify the standardized fundus image into at least two image quality grades including qualified and unqualified. Thereby, the quality of the standardized fundus images can be preliminarily filtered quickly to acquire qualified fundus images. In some examples, the qualified fundus image may be a standardized fundus image with a qualified image quality grade. Thus, a qualified fundus image can be obtained. However, the examples of the present disclosure are not limited thereto, in other examples, the standardized fundus images may be classified in more precise way in the preliminary filtering. The detailed description may refer to the relevant description of step S130, which will not be repeated here.

According to the invention as defined in claim 15, in the data preparation module 340, the target fundus image set includes the data set to be calibrated 210, the gold-standard data set 220, and the self-consistency determination data set 230. According to the invention as defined in claim 15, the data set to be calibrated 210 includes a plurality of qualified fundus images. According to the invention as defined in claim 15, gold-standard data set 220 includes a first preset number of gold-standard fundus images. The gold-standard fundus image is a fundus image for which correct annotation results are known. According to the invention as defined in claim 15, the self-consistency determination data set 230 consists of images in the data set to be calibrated 210. The number of images in the self-consistency determination data set 230 is at least one. Each image of the target fundus image set 200 is taken as each target fundus image. The detailed description may refer to the relevant description of step S140, which will not be repeated here.

According to the invention as defined in claim 15, in the annotation module 350, the doctor annotation results 130 for each image in the target fundus image set 200 includes at least one determination result. According to the invention as defined in claim 15, the determination result includes disease information which is of no abnormality or a disease. In some examples, the disease may include at least one of diabetic retinopathy, hypertensive retinopathy, glaucoma, retinal vein occlusion, retinal artery occlusion, age-related macular degeneration, high myopia macular degeneration, retinal detachment, optic nerve disease, congenital abnormalities of disc development. Thereby, at least one disease can be annotated. In some examples, in the annotation, the image quality of each image in the target fundus image set may be further classified into five image quality grades including very good, good, average, poor, and very poor. In this case, the final annotation result 150 may subsequently be determined in conjunction with a more precise image quality grade. The detailed description may refer to the relevant description of step S150, which will not be repeated here.

According to the invention as defined in claim 15, in the evaluation module 360, the doctor annotation result 130 of the first annotation doctor A meeting the self-consistency and gold-standard consistency requirements is obtained and taken as the target annotation result 140. In some examples, the preset condition may be a doctor annotation result 130 of a first annotation doctor A having a self-consistency greater than a self-consistency threshold value and a gold-standard consistency greater than a gold-standard consistency threshold value. Thus, the preset condition can be determined based on the self-consistency threshold value and the gold-standard consistency threshold value. In some examples, the preset condition may be $d_{self} \leq D$ and $d_{gold} \leq D$, wherein $d_{self}$ is a self-evaluation index based on self-consistency, $d_{gold}$ is a gold-standard evaluation index based on gold-standard consistency, and $D$ is an evaluation index threshold value. In some examples, $D \leq 5\%$. Thus, the preset condition can be determined based on the evaluation index threshold value. In some examples, the doctor annotation result 130 of the first annotation doctor A that does not meet the preset condition may be re-annotated by the second annotation doctor B for each image in the target fundus image set 200 until the doctor annotation result 130 meeting the preset condition is obtained as the target annotation result 140. The detailed description may refer to the relevant description of step S160, which will not be repeated here.

In some examples, in the evaluation module 360, the self-evaluation index $d_{self}$ may satisfy the formula: $d_{self} = |J_{self} - \kappa_{self}|/\kappa_{self} \times 100\%$, wherein $J_{self} = SE_{self} + SP_{self} - 1$, $SE_{self}$ is the sensitivity of the first annotation doctor A obtained based on the two groups of annotation results for evaluating self-consistency and $SP_{self}$ is the specificity of the first annotation doctor A obtained based on the two groups of annotation results for evaluating self-consistency, $\kappa_{self}$ is the self-consistency of the first annotation doctor A. In some examples, any one group of the two groups of annotation results used to evaluate self-consistency may be taken as a gold-standard to evaluate the other group to obtain the sensitivity and specificity of the first annotation doctor A.

In some examples, in the evaluation module 360, the gold-standard evaluation index $d_{gold}$ may satisfy the formula: $d_{gold} = |J_{gold} - \kappa_{gold}|/\kappa_{gold} \times 100\%$, wherein $J_{gold} = SE_{gold} + SP_{gold} - 1$, $SE_{gold}$ is the sensitivity of the first annotation doctor A obtained based on the two groups of annotation results for assessing the gold-standard consistency, $SP_{gold}$ is the specificity of the first annotation doctor A obtained based on the two groups of annotation results for assessing the gold-standard consistency, $\kappa_{gold}$ is the gold-standard consistency of the first annotation doctor A. In some examples, the first of the two groups of annotation results used to evaluate gold-standard consistency may be taken as the gold-standard to evaluate the second group of annotation results to obtain the sensitivity and specificity of the first annotation doctor A.

According to the invention as defined in claim 15, in calculating self-consistency, the evaluation module 360 obtains two groups of doctor annotation results 130 for each image in the self-consistency determination data set and for images in the data set to be calibrated 210 that are repeated with respective image in the self-consistency determination data set 230. Self-consistency is obtained by taking any one of the two groups of annotation results as a first group of annotation results and the other set as a second group of annotation results and evaluating using a self-consistency determination and evaluation method. Thus, the self-consistency of each first annotation doctor A can be calculated based on the self-consistency determination method. The detailed description may refer to the relevant description of step S160, which will not be repeated here.

According to the invention as defined in claim 15, in calculating the gold-standard consistency, the evaluation module 360 takes the correct annotation result for the gold-standard data set 220 as the first group of annotation results, that is, the annotation result for the gold-standard fundus image is taken as the first group of annotation results, and the doctor annotation result 130 for each image in the gold-standard data set 220 is taken as the second group of annotation results. Self-consistency is obtained based on the first group of annotation results and the second group of annotation results and evaluated using a gold-standard consistency determination evaluation method. Thus, the self-consistency of each first annotation doctor A can be calculated on the basis of the gold-standard consistency determination method. The detailed description may refer to the relevant description of step S160, which will not be repeated here.

In some examples, the self-consistency determination method in the evaluation module 360 may be using a quadratic weighted kappa coefficient to calculate the disease self-consistency for each of the first annotation doctors A determining each of the diseases. In some examples, the quadratic weighted kappa coefficient for a single disease may be

$$\kappa = 1 - \frac{\sum_{i,j} W_{ij} X_{ij}}{\sum_{i,j} W_{ij} E_{ij}}$$

Wherein $W_{ij}$ may represent a quadratic weighting coefficient, $X_{ij}$ may represent the number of target fundus images in which the determination result in the first group of annotation results is $i$ and the determination result in the second group of annotation results is $j$, $E_{ij}$ may represent the expected number of target fundus images in which the determination result in the first group of annotation results is i and the determination result in the second group of annotation results is $j$. In some examples, when $i$ is not equal to $j$, $E_{ij}$ may be zero. In some examples, a quadratic weighting factor $W_{ij}$ may be set as needed to highlight the importance of a certain determination result. Thus, it is able to check consistency between the first group of annotation results and the second group of annotation results. In some examples, in the self-consistency determination method, each disease self-consistency may be weighted to calculate the self-consistency of each first annotation doctor A. The detailed description may refer to the relevant description of step S160, which will not be repeated here.

In some examples, the gold-standard consistency determination method in the evaluation module 360 may be using a quadratic weighted kappa coefficient to calculate the disease gold-standard consistency for each of the first annotation doctors A to determine each disease. In some examples, each disease gold-standard consistency may be weighted to calculate a gold-standard consistency for each first annotation doctor A. Thus, the self-consistency of each first annotation doctor A can be calculated on the basis of the gold-standard consistency determination method. The detailed description may refer to the relevant description of step S160, which will not be repeated here.

In some examples, the evaluation module 360 may analyze the target self-consistency and target gold-standard consistency of different threshold value annotation doctors and determine the self-consistency threshold value and the gold-standard consistency threshold value in the manner of abnormality detection. Thus, the self-consistency threshold value and the gold-standard consistency threshold value can be determined. In some examples, the abnormality detection of the self-consistency threshold value may be performed in such a way as to obtain target self-consistency of different threshold value annotation doctors and to calculate a mean self-consistency $\mu_0$ and a variance self-consistency $\sigma_0$, and under the assumption that the target self-consistency satisfies a Gaussian distribution, the self-consistency threshold value may be $\mu_0$-1.96$\times\sigma_0$. In some examples, the self-consistency threshold value may be 0.7977. In some examples, the abnormality detection of the gold-standard consistency threshold value may be performed by obtaining target gold-standard consistency of annotation doctors with different threshold values and calculating a gold-standard consistency mean $\mu_1$ and a gold-standard consistency variance $\sigma_1$, and under the assumption that the target gold-standard consistency satisfies a Gaussian distribution, the gold-standard consistency threshold value may be $\mu_1$-1.96$\times\sigma_1$. In some examples, the gold-standard consistency threshold value may be 0.6235. The detailed description may refer to the relevant description of step S160, which will not be repeated here.

In some examples, absolute majority voting is used to compare each annotation result of each target fundus image in the plurality of groups of target annotation results 140 to determine a final annotation result 150 for each target fundus image. Specifically, when absolute majority voting is used to compare individual annotation result, the annotation results will be accepted as part of the final annotation result 150 if more than half of the annotation results are consistent(i.e., more than half of the valid votes are required to be accepted). In some examples, if the final annotation result 150 cannot be determined (i.e., the number of valid votes is not more than half), the target fundus image is annotated as a difficult fundus image. In some examples, difficult fundus images may be annotated and arbitrated to obtain a final annotation result 150. Thus, the final annotation result 150 can be obtained based on the absolute majority voting method. The detailed description may refer to the relevant description of step S170, and will not be repeated here.

However, the examples of the present disclosure are not limited hereto, in other examples, gathering may be comparing respective annotation results in the plurality of groups of target annotation results 140 for respective target fundus images. In some examples, in the case that the respective annotation results are consistent, the annotation results may be taken as the final annotation result 150 for the target fundus image. In some examples, in the case that the plurality of annotation results are inconsistent, if the plurality of annotation results simultaneously include the same determination result and only one annotation result includes a determination result which is not identified in other annotation results, the target fundus image may be annotated as a fundus image to be quality controlled, otherwise, the target fundus image may be annotated as a difficult fundus image. In this case, by comparing the respective annotation results of the respective target fundus images in the plurality of groups of target annotation results 140, the target fundus image can be classified into a target fundus image having a final annotation result 150, a fundus image to be quality-controlled, and a difficult fundus image, and the final annotation result 150 can be obtained. In some examples, quality control may be performed on the fundus images to be quality controlled. In some examples, if it is determined that an unidentified determination result does not exist, the same determination result may be taken as the final annotation result 150. In some examples, if it is determined that an unidentified determination result exists, the fundus image to be quality-controlled may be annotated as a difficult fundus image. In some examples, difficult fundus images may be annotated and arbitrated to obtain a final annotation result 150. Thus, the final annotation result 150 of the difficult fundus image can be obtained. The detailed description may refer to the relevant description of step S170, and will not be repeated here.

Although the present disclosure has been particularly shown and described with reference to the accompanying drawings and examples, it is to be understood that the disclosure is not limited in any manner by the foregoing description. Aspects, embodiments and examples disclosed herein which do not fall within the scope of the appended claims do not form part of the invention and are merely provided for illustrative purposes.

## Claims

1. A quality control method for data annotation on a fundus image, **characterized by** including:

   Acquiring, by an acquisition module (310), a plurality of fundus images;
   performing, by a standardization processing module (320), standardization processing on each of the plurality of fundus images to obtain a plurality of standardized fundus images;
   performing, by a preliminary filtering module (330), preliminary filtering on quality of each of the plurality of standardized fundus images to obtain a plurality of qualified fundus images;
   preparing, by a data preparation module (340), a target fundus image set (200), the target fundus image set (200) includes a data set to be calibrated (210) including the plurality of qualified fundus images, a gold-standard data set (220) including a first preset number of gold-standard fundus images with a known correct annotation result, and a self-consistency determination data set (230) composed of at least one image in the data set to be calibrated (210), and taking each image of the target fundus image set (200) as a respective target fundus image;
   annotating, by an annotation module (350), respective images of the target fundus image set (200) by a plurality of first annotation doctors (A) respectively to obtain a plurality of groups of doctor annotation results (130), the doctor annotation results (130) include at least one determination result, the determination result at least includes disease information of no obvious abnormality or of a disease;
   calculating, by an evaluation module (360), self-consistency and gold-standard consistency of the corresponding first annotation doctors (A) on the basis of the doctor annotation results (130) to acquire the doctor annotation results (130) of the first annotation doctors (A) satisfying a preset condition as target annotation results (140), obtaining the self-consistency by taking any one of two groups of annotation results of the doctor annotation result (130) of each image in the self-consistency determination data set (230) and the doctor annotation result (130) of an image, which is repeated with respective image in the self-consistency determination data set (230), in the data set to be calibrated (210) as a first group of annotation results and taking the other group as a second group of annotation results and performing evaluation using a self-consistency determination and evaluation method, acquiring the gold-standard consistency by taking the correct annotation result of the gold-standard data set (220) as a first group of annotation results and the doctor annotation result (130) of each image in the gold-standard data set (220) as a second group of annotation results and using a gold-standard consistency determination and evaluation method;

gathering, by a gathering module (370), a plurality of sets of the target annotation results (140) to obtain a final annotation result (150).

2. The quality control method according to claim 1, **characterized in that**:
the preset condition is that the self-consistency is greater than a self-consistency threshold value and the gold-standard consistency is greater than a gold-standard consistency threshold value.

3. The quality control method according to claim 1, **characterized in that**:
the doctor annotation result (130) of the first annotation doctor (A) which does not meet the preset condition is re-annotated by the second annotation doctor (B) on each image in the target fundus image set (200) until the doctor annotation result (130) meeting the preset condition is obtained as the target annotation result (140).

4. The quality control method according to claim 1, **characterized in that**:

the self-consistency determination method is to calculate a disease self-consistency of each first annotation doctor (A) determining each disease by using a quadratic weighted kappa coefficient and to weight each disease self-consistency so as to calculate the self-consistency of each first annotation doctor (A);
the gold-standard consistency determination method is to calculate the gold-standard consistency of a disease of each first annotation doctor (A) determining each disease using a quadratic weighted kappa coefficient and to weight the gold-standard consistency of the disease so as to calculate the gold-standard consistency of each first annotation doctor (A).

5. The quality control method according to claim 4, **characterized in that**:

the quadratic weighted kappa coefficient $\kappa$ is

$$\kappa = 1 - \frac{\sum_{i,j} W_{ij} X_{ij}}{\sum_{i,j} W_{ij} E_{ij}},$$

wherein $W_{ij}$ represents a quadratic weighting coefficient, $X_{ij}$ represents a number of the target fundus images for which the determination result in the first group of annotation results is $i$ and the determination result in the second group of annotation results is $j$, and $E_{ij}$ represents an expected number of the target fundus images for which the determination result in the first group of annotation results is $i$ and the determination result in the second group of annotation results is $j$.

6. The quality control method according to claim 2, **characterized in that**:
target self-consistency and target gold-standard consistency of doctors with different threshold value annotation are analyzed and abnormality detection is used to determine the self-consistency threshold value and the gold-standard consistency threshold value.

7. The quality control method according to claim 6, **characterized in that**:
the abnormality detection is to acquire the target self-consistency of the doctors with different threshold value annotation and calculate a self-consistency mean value $\mu_0$ and a self-consistency variance $\sigma_0$, under the assumption that the target self-consistency satisfies a Gaussian distribution, the self-consistency threshold value is $\mu_0 - 1.96 \times \sigma_0$, and to acquire the target gold-standard consistency of the doctors with different threshold value annotation and calculate a gold-standard consistency mean value $\mu_1$ and a gold-standard consistency variance $\sigma_1$, under the assumption that the target gold-standard consistency satisfies a Gaussian distribution, the gold-standard consistency threshold value is $\mu_1 - 1.96 \times \sigma_1$.

8. The quality control method according to claim 1, **characterized in that**:

the gathering is to compare each annotation result of each the target fundus image in a plurality of groups of the target annotation results (140) using an absolute majority voting method to determine the final annotation result (150) of each the target fundus image, and if the final annotation result (150) is not able to be determined, the target fundus image is annotated as a difficult fundus image; and
the difficult fundus image is annotated and arbi-

9. The quality control method according to claim 1, **characterized in that**:
The gathering is to compare each annotation result of each target fundus image in the plurality of groups of the target annotation results (140), in the case that each annotation result is consistent, taking the annotation result as the final annotation result (150) of the target fundus image, while in the case that the plurality of annotation results are inconsistent, if the plurality of annotation results simultaneously include a same determination result and only one annotation result includes a determination result which is not identified in the other annotation results, the target fundus image is annotated as a fundus image to be quality-controlled, otherwise, the target fundus image is annotated as a difficult fundus image; quality control is performed on the fundus image to be quality-controlled and the final annotation result (150) is obtained, and the difficult fundus image is annotated and arbitrated so as to obtain the final annotation result (150).

10. The quality control method according to claim 1, **characterized in that**:
In the preliminary filtering, the quality of the standardized fundus image may be determined by a plurality of first annotation doctors (A) to classify the standardized fundus image into a plurality of image quality grades, the qualified fundus image is the standardized fundus image of which the image quality grade is qualified.

11. The quality control method according to claim 10, **characterized in that**:
the standardized fundus image are ranked based on factors that affect the quality of the fundus image, the factors affecting the quality of the fundus image include at least one of location at which the fundus image was taken, exposure, and definition.

12. The quality control method according to claim 1, **characterized in that**:
the disease comprises at least one of diabetic retinopathy, hypertensive retinopathy, glaucoma, retinal vein occlusion, retinal artery occlusion, age-related macular degeneration, high myopia macular degeneration, retinal detachment, optic nerve disease, congenital abnormalities of disc development.

13. The quality control method according to claim 1, **characterized in that**:

the preset condition is $d_{self} \leq D$ and $d_{gold} \leq D$, wherein $d_{self}$ is a self-evaluation index based on the self-consistency, $d_{gold}$ is a gold-standard evaluation index based on the gold-standard consistency, and $D$ is an evaluation index threshold value;
the self-evaluation index $d_{self}$ satisfies the formula: $d_{self} = |J_{self} - \kappa_{self}|/\kappa_{self} \times 100\%$, wherein $J_{self} = SE_{self} + SP_{self} - 1$, $SE_{self}$ is sensitivity of the first annotation doctor (A) obtained based on the two groups of annotation results for evaluating the self-consistency, $SP_{self}$ is specificity of the first annotation doctor (A) obtained based on the two groups of annotation results for evaluating the self-consistency, $\kappa_{self}$ is the self-consistency of the first annotation doctor (A);
the gold-standard evaluation index $d_{gold}$ satisfies the formula: $d_{gold} = |J_{gold} - \kappa_{gold}|/\kappa_{gold} \times 100\%$, wherein $J_{gold} = SE_{gold} + SP_{gold} - 1$, $SE_{gold}$ is sensitivity of the first annotation doctor (A) obtained on the basis of the two groups of annotation results for evaluating the gold-standard consistency, and $SP_{gold}$ is specificity of the first annotation doctor (A) obtained on the basis of the two groups of annotation results for evaluating the gold-standard consistency, $\kappa_{gold}$ is the gold-standard consistency of the first annotation doctor (A).

14. The quality control method according to claim 1, **characterized in that**:
the standardization processing includes at least one of dividing the fundus images per the patient, unifying a name format of the fundus images, filtering out a non-fundus image, unifying a picture format of the fundus images, and unifying a background of the fundus images.

15. A quality control system (300) for data annotation on a fundus image, including:

an acquisition module (310), configured to acquire a plurality of fundus images;
a standardization processing module (320), configured to perform standardization processing on each of the plurality of fundus images to obtain a plurality of standardized fundus images;
a preliminary filtering module (330), configured to perform preliminary filtering on quality of each of the standardized fundus images to obtain a plurality of qualified fundus images;
a data preparation module (340), configured to prepare a target fundus image set (200), the target fundus image set (200) includes a data set to be calibrated (210) including the plurality of qualified fundus images, a gold-standard data set (220) including a first preset number of gold-standard fundus images with a known correct annotation result, and a self-consistency determination data set (230) composed of at least one image in the data set to be calibrated (210), each image of the target fundus image set (200) is taken as each target fundus image; an annotation module (350), configured to acquire a plurality of groups of doctor annotation results (130) by a plurality of first annotation doctors (A) respectively annotating each image in the target fundus image set (200), the doctor annotation results (130) include at least one determination result, the determination result at least includes disease information of no obvious abnormality or of a disease; and an evaluation module (360), configured to calculate a self-consistency and a gold-standard consistency of a corresponding first annotation doctor (A) based on the doctor annotation result (130) so as to obtain the doctor annotation result (130) of the first annotation doctor (A) satisfying a preset condition as a target annotation result (140), the self-consistency is obtained by taking any one of two groups of annotation results of the doctor annotation result (130) of each image in the self-consistency determination data set (230) and the doctor annotation result (130) of an image, which is repeated with respective image in the self-consistency determination data set (230), in the data set to be calibrated (210) as a first group of annotation results and the other group as a second group of annotation results and performing evaluation using a self-consistency determination and evaluation method, the gold-standard consistency is obtained by taking the correct annotation result of the gold-standard data set (220) as a first group of annotation results and the doctor annotation result (130) of each image in the gold-standard data set (220) as a second group of annotation results and using a gold-standard consistency determination and evaluation method; a gathering module (370), configured to gather the plurality of groups of the target annotation results (140) to obtain a final annotation result (150).

**Patentansprüche**

1. Qualitätskontrollverfahren für die Datenannotation auf einem Fundusbild, **dadurch gekennzeichnet, dass** es Folgendes umfasst:

Erfassen einer Vielzahl von Fundusbildern mittels eines Erfassungsmoduls (310); Durchführen einer Standardisierungsverarbeitung an jedem der Vielzahl von Fundusbildern mittels eines Standardisierungsverarbeitungsmoduls (320), um eine Vielzahl standardisierter Fundusbilder zu erhalten; Durchführen einer Vorfilterung der Qualität jedes der Vielzahl von standardisierten Fundusbilder mittels eines Vorfilterungsmoduls (330), um eine Vielzahl qualifizierter Fundusbilder zu erhalten; Vorbereiten eines Zielfundusbildsatzes (200) mittels eines Datenvorbereitungsmoduls (340), wobei der Zielfundusbildsatz (200) einen zu kalibrierenden Datensatz (210), der die Vielzahl von qualifizierten Fundusbildern enthält, einen Goldstandarddatensatz (220), der eine erste voreingestellte Anzahl von Goldstandardfundusbildern mit einem bekannten korrekten Annotationsergebnis enthält, und einen Selbstkonsistenzbestimmungsdatensatz (230), der aus mindestens einem Bild in dem zu kalibrierenden Datensatz (210) besteht, und wobei jedes Bild des Zielfundusbildsatzes (200) als ein jeweiliges Zielfundusbild verwendet wird; Annotation mittels eines Annotationsmoduls (350) jeweiliger Bilder des Zielfundusbildsatzes (200) durch eine Vielzahl von ersten Annotationsärzten (A), um eine Vielzahl von Gruppen von Arztannotationsergebnissen (130) zu erhalten, wobei die Arztannotationsergebnisse (130) mindestens ein Bestimmungsergebnis enthalten, wobei das Bestimmungsergebnis mindestens Krankheitsinformationen über das Fehlen einer offensichtlichen Anomalie oder einer Krankheit enthält; Berechnen mittels eines Auswertungsmoduls (360) der Selbstkonsistenz und der Goldstandardkonsistenz der entsprechenden ersten Annotationsärzte (A) auf Grundlage der Arztannotationsergebnisse (130), um die Arztannotationsergebnisse (130) der ersten Annotationsärzte (A), die eine vorgegebene Bedingung erfüllen, als Zielannotationsergebnisse (140) zu erhalten, Erhalten der Selbstkonsistenz, indem eine beliebige von zwei Gruppen von Annotationsergebnissen des Arztannotationsergebnisses (130) jedes Bildes in dem Selbstkonsistenzbestimmungsdatensatz (230) und des Arztannotationsergebnisses (130) eines Bildes verwendet wird, das mit dem jeweiligen Bild in dem Selbstkonsistenzbestimmungsdatensatz (230) wiederholt wird, in dem zu kalibrierenden Datensatz (210) als eine erste Gruppe von Annotationsergebnissen verwendet wird und die andere Gruppe als eine zweite Gruppe von Annotationsergebnissen verwendet wird, und das Durchführen einer Auswertung unter Verwendung eines Selbstkonsistenzbestimmungs- und Auswertungsverfahrens, wobei die Goldstandardkonsistenz durch die Verwendung des korrekten Annotationsergebnisses des Goldstandarddatensatzes (220) als eine erste Gruppe von Annotationsergebnissen und des Arztannotationsergebnisses (130) jedes Bildes in dem Goldstandarddatensatz (220) als eine zweite Gruppe von Annotationsergebnissen und unter Verwendung eines Verfahrens zur Bestimmung der Goldstandardkonsistenz und Auswertung ermittelt wird;

Sammeln einer Vielzahl von Sätzen der Zielannotationssergebnisse (140) mittels eines Sammelmoduls (370), um ein endgültiges Annotationsergebnis (150) zu erhalten.

2. Qualitätskontrollverfahren nach Anspruch 1, **dadurch gekennzeichnet, dass**:

die vorgegebene Bedingung ist, dass die Selbstkonsistenz größer ist als ein Schwellenwert für die Selbstkonsistenz und die Goldstandardkonsistenz größer ist als ein Schwellenwert für die Goldstandardkonsistenz.

3. Qualitätskontrollverfahren nach Anspruch 1, **dadurch gekennzeichnet, dass**:

das Arztannotationsergebnis (130) des ersten Annotationsarztes (A), das die vorgegebene Bedingung nicht erfüllt, von dem zweiten Annotationsarzt (B) auf jedem Bild des Zielfundusbildsatzes (200) neu annotiert wird, bis das Arztannotationsergebnis (130), das die vorgegebene Bedingung erfüllt, als Zielannotationsergebnis (140) erhalten wird.

4. Qualitätskontrollverfahren nach Anspruch 1, **dadurch gekennzeichnet, dass**:

das Verfahren zur Bestimmung der Selbstkonsistenz darin besteht, eine Krankheitsselbstkonsistenz jedes ersten Annotationsarztes (A) bei der Bestimmung jeder Krankheit mithilfe eines quadratisch gewichteten Kappa-Koeffizienten zu berechnen und jede Krankheitsselbstkonsistenz zu gewichten, um die Selbstkonsistenz jedes ersten Annotationsarztes (A) zu berechnen; das Verfahren zur Bestimmung der Goldstandardkonsistenz darin besteht, die Goldstandardkonsistenz einer Krankheit jedes ersten Annotationsarztes (A) bei der Bestimmung jeder Krankheit mithilfe eines quadratisch gewichteten Kappa-Koeffizienten zu berechnen und die Goldstandardkonsistenz der Krankheit zu gewichten, um die Goldstandardkonsistenz jedes ersten Annotationsarztes (A) zu berechnen.

5. Qualitätskontrollverfahren nach Anspruch 4, **dadurch gekennzeichnet, dass**:

der quadratisch gewichtete Kappa-Koeffizient K wie folgt lautet

$$\kappa = 1 - \frac{\sum_{i,j} W_{ij} X_{ij}}{\sum_{i,j} W_{ij} E_{ij}},$$

wobei $W_{ij}$ einen quadratischen Gewichtungskoeffizienten angibt, $X_{ij}$ eine Anzahl der Zielfundusbilder angibt, für die das Bestimmungsergebnis in der ersten Gruppe von Annotationsergebnissen $i$ ist und das Bestimmungsergebnis in der zweiten Gruppe von Annotationsergebnissen $j$ ist, und $E_{ij}$ eine erwartete Anzahl der Zielfundusbilder angibt, für die das Bestimmungsergebnis in der ersten Gruppe von Annotationsergebnissen $i$ und das Bestimmungsergebnis in der zweiten Gruppe von Annotationsergebnissen $j$ ist.

6. Qualitätskontrollverfahren nach Anspruch 2, **dadurch gekennzeichnet, dass**:

die Zielselbstkonsistenz und die Zielgoldstandardkonsistenz von Ärzten mit unterschiedlichen Schwellenwertannotationen analysiert werden, und eine Anomalieerkennung verwendet wird, um den Schwellenwert für die Selbstkonsistenz und den Schwellenwert für die Goldstandardkonsistenz zu bestimmen.

7. Qualitätskontrollverfahren nach Anspruch 6, **dadurch gekennzeichnet, dass**:

die Anomalieerkennung darin besteht, die Zielselbstkonsistenz der Ärzte mit verschiedenen Schwellenwertannotationen zu erfassen und einen Mittelwert $\mu_0$ und eine Varianz $\sigma_0$ der Selbstkonsistenz zu berechnen, wobei angenommen wird, dass die Zielselbstkonsistenz einer Gaußschen Verteilung folgt, der Selbstkonsistenzschwellenwert $\mu_0$-1.96 x $\sigma_0$ beträgt, und die Zielgoldstandardkonsistenz der Ärzte mit verschiedenen Schwellenwertannotationen zu erfassen und einen Mittelwert $\mu_1$ und eine Varianz $\sigma_1$ der Goldstandardkonsistenz zu berechnen, wobei angenommen wird, dass die Zielgoldstandardkonsistenz einer Gaußschen Verteilung folgt, und der Schwellenwert für die Goldstandardkonsistenz $\mu_1$-1.96 x $\sigma_1$ beträgt.

8. Qualitätskontrollverfahren nach Anspruch 1, **dadurch gekennzeichnet, dass**:

das Sammeln darin besteht, jedes Annotationsergebnis jedes Zielfundusbildes in einer Vielzahl von Gruppen der Zielannotationsergebnisse (140) unter Verwendung eines Abstimmungsverfahrens mit absoluter Mehrheit zu vergleichen, um das endgültige Annotationsergebnis (150) jedes Zielfundusbildes zu bestimmen, und wenn das endgültige Annotationsergebnis (150) nicht bestimmt werden kann, das Zielfundusbild als ein schwieriges Fundusbild annotiert wird; und

das schwierige Fundusbild annotiert und durch eine Schiedsinstanz beurteilt wird, um das endgültige Annotationsergebnis (150) zu erhalten.

9. Qualitätskontrollverfahren nach Anspruch 1, **dadurch gekennzeichnet, dass**:

das Sammeln darin besteht, jedes Annotationsergebnis jedes Zielfundusbildes in der Vielzahl von Gruppen der Zielannotationsergebnisse (140) zu vergleichen, wobei in dem Fall, dass jedes Annotationsergebnis konsistent ist, das Annotationsergebnis als das endgültige Annotationsergebnis (150) des Zielfundusbildes verwendet wird, während in dem Fall, dass die Vielzahl von Annotationsergebnissen inkonsistent sind, wenn die mehreren Annotationsergebnisse gleichzeitig ein gleiches Bestimmungsergebnis enthalten und nur ein Annotationsergebnis ein Bestimmungsergebnis enthält, das in den anderen Annotationsergebnissen nicht vorkommt, das Zielfundusbild als ein Fundusbild annotiert wird, das einer Qualitätskontrolle zu unterziehen ist, wobei das Zielfundusbild anderenfalls als ein schwieriges Fundusbild annotiert wird; die Qualitätskontrolle an dem einer Qualitätskontrolle zu unterziehenden Fundusbild durchgeführt wird und das endgültige Annotationsergebnis (150) erhalten wird, und das schwierige Fundusbild annotiert und durch eine Schiedsinstanz beurteilt wird, um das endgültige Annotationsergebnis (150) zu erhalten.

10. Qualitätskontrollverfahren nach Anspruch 1, **dadurch gekennzeichnet, dass**:

bei der Vorfilterung die Qualität des standardisierten Fundusbildes durch eine Vielzahl von ersten Annotationsärzten (A) bestimmt werden kann, um das standardisierte Fundusbild in eine Vielzahl von Bildqualitätsstufen einzuteilen, wobei das qualifizierte Fundusbild das standardisierte Fundusbild ist, dessen Bildqualitätsstufe qualifiziert ist.

11. Qualitätskontrollverfahren nach Anspruch 10, **dadurch gekennzeichnet, dass**:

die standardisierten Fundusbilder anhand von Faktoren, die die Qualität des Fundusbildes beeinflussen, eingestuft werden, wobei zu diesen Faktoren mindestens der Aufnahmeort, die Belichtung und die Auflösung gehören.

12. Qualitätskontrollverfahren nach Anspruch 1, **dadurch gekennzeichnet, dass**:

die Krankheit mindestens eine der folgenden Krankheiten umfasst: diabetische Retinopathie, hypertensive Retinopathie, Glaukom, Netzhautvenenverschluss, Netzhautarterienverschluss, altersbedingte Makuladegeneration, Makuladegeneration bei hoher Myopie, Netzhautablösung, Erkrankung des Sehnervs, angeborene Anomalien der Papillenentwicklung.

13. Qualitätskontrollverfahren nach Anspruch 1, **dadurch gekennzeichnet, dass**:

die vorgegebene Bedingung ist, dass $d_{self} \leq D$ und $d_{gold} \leq D$, wobei $d_{self}$ ein Selbstbewertungsindex auf Grundlage der Selbstkonsistenz ist, $d_{gold}$ ein Goldstandardbewertungsindex auf Grundlage der Goldstandardkonsistenz ist und ein Schwellenwert für den Bewertungsindex ist;

der Selbstbewertungsindex $d_{self}$ die Formel: $d_{self} = |J_{self} - K_{self}| / K_{self} \times 100\%$ erfüllt, wobei $J_{self} = SE_{self} + SP_{self} - 1$, $SE_{self}$ die Sensitivität des ersten Annotationsarztes (A) ist, die auf Grundlage der beiden Gruppen von Annotationsergebnissen für die Bewertung der Selbstkonsistenz erhalten wird, $SP_{self}$ die Spezifizität des ersten Annotationsarztes (A) ist, die auf Grundlage der beiden Gruppen von Annotationsergebnissen zur Bewertung der Selbstkonsistenz erhalten wird, $K_{self}$ die Selbstkonsistenz des ersten Annotationsarztes (A) ist;

der Goldstandardbewertungsindex $d_{gold}$ die Formel: $d_{gold} = |J_{gold} - K_{gold}| / K_{gold} \times 100\%$ erfüllt, wobei $j_{gold} = SE_{gold} + SP_{gold} - 1$, $SE_{gold}$ die Sensitivität des ersten Annotationsarztes (A) ist, die auf Grundlage der beiden Gruppen von Annotationsergebnissen für die Bewertung der Goldstandardkonsistenz erhalten wird, **und** $SP_{gold}$ die Spezifität des ersten Annotationsarztes (A) ist, die auf Grundlage der beiden Gruppen von Annotationsergebnissen zur Bewertung der Goldstandardkonsistenz erhalten wird, $K_{gold}$ die Goldstandardkonsistenz des ersten Annotationsarztes (A) ist.

14. Qualitätskontrollverfahren nach Anspruch 1, **dadurch gekennzeichnet, dass**:

die Standardisierungsverarbeitung mindestens einen der folgenden Schritte umfasst: Aufteilung der Fundusbilder nach Patient, Vereinheitlichung eines Namensformats der Fundusbilder, Herausfiltern eines Nicht-Fundusbildes, Vereinheitlichung eines Bildformats der Fundusbilder und Vereinheitlichung eines Hintergrunds der Fundusbilder.

15. Qualitätskontrollsystem (300) für die Datenannotation auf einem Fundusbild, das Folgendes umfasst:

ein Erfassungsmodul (310), das so konfiguriert ist, dass es eine Vielzahl von Fundusbildern erfasst;

ein Standardisierungsverarbeitungsmodul (320), das so konfiguriert ist, dass es eine Standardisierungsverarbeitung an jedem der Vielzahl von Fundusbildern durchführt, um eine Vielzahl von standardisierten Fundusbildern zu erhalten; ein Vorfilterungsmodul (330), das so konfiguriert ist, dass es eine Vorfilterung der Qualität jedes der standardisierten Fundusbilder durchführt, um eine Vielzahl von qualifizierten Fundusbildern zu erhalten;

ein Datenvorbereitungsmodul (340), das so konfiguriert ist, dass es einen Zielfundusbildsatz (200) vorbereitet, wobei der Zielfundusbildsatz (200) einen zu kalibrierenden Datensatz (210) enthält, der die Vielzahl von qualifizierten Fundusbildern umfasst, einen Goldstandarddatensatz (220), der eine erste vorgegebene Anzahl von Goldstandardfundusbildern mit einem bekannten korrekten Annotationsergebnis enthält, und einen Selbstkonsistenzbestimmungsdatensatz (230), der aus mindestens einem Bild in dem zu kalibrierenden Datensatz (210) besteht, wobei jedes Bild des Zielgundusbildsatzes (200) als ein Zielfundusbild verwendet wird;

ein Annotationsmodul (350), das so konfiguriert ist, dass es eine Vielzahl von Gruppen von Arztannotationsergebnissen (130) durch eine Vielzahl von ersten Annotationsärzten (A) erfasst, die jeweils jedes Bild in dem Zielfundusbildsatz (200) annotieren, wobei die Arztannotationsergebnisse (130) mindestens ein Bestimmungsergebnis enthalten, und das Bestimmungsergebnis mindestens Krankheitsinformationen über das Fehlen einer offensichtlichen Anomalie oder einer Krankheit enthält; und

ein Auswertungsmodul (360), das so konfiguriert ist, dass es eine Selbstkonsistenz und eine Goldstandardkonsistenz eines entsprechenden ersten Annotationsarztes (A) auf Grundlage des Arztannotationsergebnisses (130) berechnet, um das Arztannotationsergebnis (130) des ersten Annotationsarztes (A), das eine vorgegebene Bedingung als Zielannotationsergebnis (140) erfüllt, zu erhalten, wobei die Selbstkonsistenz erhalten wird, indem eine beliebige von zwei Gruppen von Annotationsergebnissen des Arztannotationsergebnisses (130) jedes Bildes in dem Selbstkonsistenzbestimmungsdatensatz (230) und des Arztannotationsergebnisses (130) eines Bildes verwendet wird, die mit dem jeweiligen Bild in dem Selbstkonsistenzbestimmungsdatensatz (230) wiederholt wird, in dem zu kalibrierenden Datensatz (210) als eine erste Gruppe von Annotationsergebnissen und die andere Gruppe als eine zweite Gruppe von Annotationsergebnissen und Durchführen einer Bewertung unter Verwendung eines Selbstkonsistenzbestimmungs- und Bewertungsverfahrens, wobei die Goldstandardkonsistenz erhalten wird, indem das korrekte Annotationsergebnis des Goldstandarddatensatzes (220) als eine erste Gruppe von Annotationsergebnissen und das Arztannotationsergebnis (130) jedes Bildes in dem Goldstandarddatensatz (220) als eine zweite Gruppe von Annotationsergebnissen verwendet wird und ein Goldstandardkonsistenzbestimmungs- und Bewertungsverfahren verwendet wird;

ein Sammelmodul (370), das so konfiguriert ist, dass es die Vielzahl von Gruppen der Zielannotationsergebnisse (140) sammelt, um ein endgültiges Annotationsergebnis (150) zu erhalten.

## Revendications

1. Procédé de contrôle qualité pour une annotation de données sur une image de fond d'œil, **caractérisé en ce qu'**il comporte :

l'acquisition, par un module d'acquisition (310), d'une pluralité d'images de fond d'œil ;
la réalisation, par un module de traitement de normalisation (320), d'un traitement de normalisation sur chacune de la pluralité d'images de fond d'œil pour obtenir une pluralité d'images de fond d'œil normalisées ;
la réalisation, par un module de filtrage préliminaire (330), d'un filtrage préliminaire sur la qualité de chacune de la pluralité d'images de fond d'œil normalisées pour obtenir une pluralité d'images de fond d'œil qualifiées ;
la préparation, par un module de préparation de données (340), d'un ensemble d'images de fond d'œil cible (200), l'ensemble d'images de fond d'œil cible (200) comporte un ensemble de données à calibrer (210) comportant la pluralité d'images de fond d'œil qualifiées, un ensemble de données d'étalon-or (220) comportant un premier nombre prédéfini d'images de fond d'œil d'étalon-or avec un résultat d'annotation correct connu, et un ensemble de données de détermination d'autocohérence (230) composé d'au moins une image dans l'ensemble de données à calibrer (210), et la prise de chaque image de l'ensemble d'images de fond d'œil cible (200) en tant qu'une image de fond d'œil cible respective ;
l'annotation, par un module d'annotation (350), d'images respectives de l'ensemble d'images de fond d'œil cible (200) par une pluralité de premiers médecins d'annotation (A) respectivement pour obtenir une pluralité de groupes de résultats d'annotation de médecin (130), les résultats d'annotation de médecin (130) comportent au moins un résultat de détermination, le résultat de la détermination comporte au moins des informations de maladie d'absence d'anomalie évidente ou d'une maladie ;

le calcul, par un module d'évaluation (360), d'une autocohérence et d'une cohérence d'étalon-or des premiers médecins d'annotation (A) correspondants en fonction des résultats d'annotation de médecin (130) pour acquérir les résultats d'annotation de médecin (130) des premiers médecins d'annotation (A) satisfaisant à une condition prédéfinie en tant que résultats d'annotation cibles (140), l'obtention de l'autocohérence par la prise de l'un quelconque des deux groupes de résultats d'annotation du résultat d'annotation de médecin (130) de chaque image dans l'ensemble de données de détermination d'autocohérence (230) et du résultat d'annotation de médecin (130) d'une image, qui est répétée avec l'image respective dans l'ensemble de données de détermination d'autocohérence (230), dans l'ensemble de données à calibrer (210) en tant que premier groupe de résultats d'annotation et par la prise de l'autre groupe en tant que second groupe de résultats d'annotation et la réalisation d'une évaluation à l'aide d'un procédé de détermination et d'évaluation d'autocohérence, l'acquisition de la cohérence d'étalon-or par la prise du résultat d'annotation correct de l'ensemble de données d'étalon-or (220) en tant que premier groupe de résultats d'annotation et du résultat d'annotation de médecin (130) de chaque image dans l'ensemble de données d'étalon-or (220) en tant que second groupe de résultats d'annotation et utilisation d'un procédé de détermination et d'évaluation de cohérence d'étalon-or ;

le rassemblement, par un module de rassemblement (370), d'une pluralité d'ensembles des résultats d'annotation cibles (140) pour obtenir un résultat d'annotation final (150).

2. Procédé de contrôle qualité selon la revendication 1, **caractérisé en ce que** :
la condition prédéfinie est que l'autocohérence est supérieure à une valeur seuil d'autocohérence et que la cohérence d'étalon-or est supérieure à une valeur seuil de cohérence d'étalon-or.

3. Procédé de contrôle qualité selon la revendication 1, **caractérisé en ce que** :
le résultat d'annotation de médecin (130) du premier médecin d'annotation (A) qui ne satisfait pas à la condition prédéfinie est réannoté par le second médecin d'annotation (B) sur chaque image dans l'ensemble d'images de fond d'œil cible (200) jusqu'à ce que le résultat d'annotation de médecin (130) qui satisfait à la condition prédéfinie soit obtenu en tant que résultat d'annotation cible (140).

4. Procédé de contrôle qualité selon la revendication 1, **caractérisé en ce que** :

le procédé de détermination d'autocohérence est de calculer une autocohérence de maladie de chaque premier médecin d'annotation (A) déterminant chaque maladie à l'aide d'un coefficient kappa pondéré quadratique et de pondérer chaque autocohérence de maladie de manière à calculer l'autocohérence de chaque premier médecin d'annotation (A) ;

le procédé de détermination de cohérence d'étalon-or est de calculer la cohérence d'étalon-or d'une maladie de chaque premier médecin d'annotation (A) déterminant chaque maladie à l'aide d'un coefficient kappa pondéré quadratique et de pondérer la cohérence d'étalon-or de la maladie de manière à calculer la cohérence d'étalon-or de chaque premier médecin d'annotation (A).

5. Procédé de contrôle qualité selon la revendication 4, **caractérisé en ce que** :

le coefficient kappa pondéré quadratique K est

$$\kappa = 1 - \frac{\sum_{i,j} W_{ij} X_{ij}}{\sum_{i,j} W_{ij} E_{ij}},$$

où $W_{ij}$ représente un coefficient de pondération quadratique, $X_{ij}$ représente un nombre d'images de fond d'œil cible pour lesquelles le résultat de détermination dans le premier groupe de résultats d'annotation est $i$ et le résultat de la détermination dans le second groupe de résultats d'annotation est $j$, et $E_{ij}$ représente un nombre attendu d'images de fond d'œil cible pour lesquelles le résultat de détermination dans le premier groupe de résultats d'annotation est $i$ et le résultat de détermination dans le second groupe de résultats d'annotation est $j$.

6. Procédé de contrôle qualité selon la revendication 2, **caractérisé en ce que** :
une autocohérence cible et la cohérence d'étalon-or cible des médecins avec une annotation de valeur seuil différente sont analysées et une détection d'anomalie est utilisée pour déterminer la valeur seuil d'autocohérence et la valeur seuil de cohérence d'étalon-or.

7. Procédé de contrôle qualité selon la revendication 6, **caractérisé en ce que** :
la détection d'anomalie est d'acquérir l'autocohérence cible des médecins avec une annotations de valeur seuil différente et calculer une valeur

moyenne d'autocohérence $\mu_0$ et une variance d'autocohérence $\sigma_0$, dans l'hypothèse où l'autocohérence cible satisfait à une distribution gaussienne, la valeur seuil d'autocohérence est $\mu_0$-1,96 x $\sigma_0$, et d'acquérir la cohérence d'étalon-or cible des médecins avec une annotation de valeur seuil différente et calculer une valeur moyenne de cohérence d'étalon-or $\mu_1$ et une variance de cohérence d'étalon-or $\sigma_1$, dans l'hypothèse où la cohérence d'étalon-or cible satisfait à une distribution gaussienne, la valeur seuil de cohérence d'étalon-or est $\mu_1$-1,96 x $\sigma_1$.

8. Procédé de contrôle qualité selon la revendication 1, **caractérisé en ce que** :

> le rassemblement est de comparer chaque résultat d'annotation de chaque image de fond d'œil cible dans une pluralité de groupes de résultats d'annotation cibles (140) à l'aide d'un procédé de vote à la majorité absolue pour déterminer le résultat d'annotation final (150) de chaque image de fond d'œil cible, et si le résultat d'annotation final (150) ne peut pas être déterminé, l'image de fond d'œil cible est annotée comme une image de fond d'œil difficile ; et l'image de fond d'œil difficile est annotée et arbitrée pour obtenir le résultat d'annotation final (150).

9. Procédé de contrôle qualité selon la revendication 1, **caractérisé en ce que** :
le rassemblement est de comparer chaque résultat d'annotation de chaque image de fond d'œil cible dans la pluralité de groupes de résultats d'annotation cibles (140), dans le cas où chaque résultat d'annotation est cohérent, prendre le résultat d'annotation en tant que résultat d'annotation final (150) de l'image de fond d'œil cible, alors que dans le cas où la pluralité de résultats d'annotation sont incohérents, si la pluralité de résultats d'annotation comporte simultanément un même résultat de détermination et qu'un seul résultat d'annotation comporte un résultat de détermination qui n'est pas identifié dans les autres résultats d'annotation, l'image de fond d'œil cible est annotée en tant qu'une image de fond d'œil devant faire l'objet d'un contrôle qualité, autrement, l'image de fond d'œil cible est annotée comme une image de fond d'œil difficile ; un contrôle qualité est réalisé sur l'image de fond d'œil devant faire l'objet d'un contrôle qualité et le résultat d'annotation final (150) est obtenu, et l'image de fond d'œil difficile est annotée et arbitrée de manière à obtenir le résultat d'annotation final (150).

10. Procédé de contrôle qualité selon la revendication 1, **caractérisé en ce que** :
dans le filtrage préliminaire, la qualité de l'image de fond d'œil normalisée peut être déterminée par une pluralité de premiers médecins d'annotation (A) pour classifier l'image de fond d'œil normalisée dans une pluralité de catégories de qualité d'image, l'image de fond d'œil qualifiée est l'image de fond d'œil normalisée dont la catégorie de qualité d'image est qualifiée.

11. Procédé de contrôle qualité selon la revendication 10, **caractérisé en ce que** :
l'image de fond d'œil normalisée sont classées en fonction de facteurs qui affectent la qualité de l'image de fond d'œil, les facteurs affectant la qualité de l'image de fond d'œil comportent au moins l'un d'un emplacement au niveau duquel l'image de fond d'œil a été prise, d'une exposition et d'une définition.

12. Procédé de contrôle qualité selon la revendication 1, **caractérisé en ce que** :
la maladie comprend au moins une rétinopathie diabétique, une rétinopathie hypertensive, un glaucome, une occlusion de la veine rétinienne, une occlusion de l'artère rétinienne, une dégénérescence maculaire liée à l'âge, une dégénérescence maculaire à forte myopie, un décollement de rétine, une maladie du nerf optique, des anomalies congénitales du développement de disque.

13. Procédé de contrôle qualité selon la revendication 1, **caractérisé en ce que** :

> la condition prédéfinie est $d_{self} \leq D$ et $d_{gold} \leq D$, dans lequel $d_{self}$ est un indice d'auto-évaluation en fonction de l'autocohérence, $d_{gold}$ est un indice d'évaluation d'étalon-or en fonction de la cohérence d'étalon-or, et est une valeur seuil d'indice d'évaluation ;
> l'indice d'auto-évaluation $d_{self}$ satisfait à la formule : $d_{self}=|J_{self}-K_{self}| / K_{self}\times100\%$, dans lequel $J_{self} = SE_{self} + SP_{self} - 1$, $SE_{self}$ est une sensibilité du premier médecin d'annotation (A) obtenue en fonction des deux groupes de résultats d'annotation pour une évaluation de l'autocohérence, $SP_{self}$ est une spécificité du premier médecin d'annotation (A) obtenue en fonction des deux groupes de résultats d'annotation pour une évaluation de l'autocohérence, $K_{self}$ est l'autocohérence du premier médecin d'annotation (A) ;
> l'indice d'évaluation d'étalon-or $d_{gold}$ satisfait à la formule : $d_{gold}=|J_{gold}-K_{gold}|/ K_{gold} \times100\%$, dans lequel $j_{gold} = SE_{gold} + SP_{gold}-1$, $SE_{gold}$ est une sensibilité du premier docteur d'annotation (A) obtenue en fonction des deux groupes de résultats d'annotation pour évaluer la cohérence d'étalon-or, et $SP_{gold}$ est une spécificité du premier docteur d'annotation (A) obtenue en fonction des deux groupes de résultats d'annotation pour évaluer la cohérence d'étalon-or,

$K_{gold}$ est la cohérence d'étalon-or du premier médecin d'annotation (A).

14. Procédé de contrôle qualité selon la revendication 1, **caractérisé en ce que** :

le traitement de normalisation comporte au moins l'un d'une division des images de fond d'œil par patient, d'une unification d'un format de nom pour les images de fond d'œil, d'une élimination par filtrage d'une image autre que du fond d'œil, d'une unification d'un format d'illustration des images de fond d'œil et d'une unification d'un arrière-plan des images de fond d'œil.

15. Système de contrôle qualité (300) pour une annotation de données sur une image de fond d'œil, comportant :

un module d'acquisition (310), conçu pour acquérir une pluralité d'images de fond d'œil ;

un module de traitement de normalisation (320), conçu pour réaliser un traitement de normalisation sur chacune de la pluralité d'images de fond d'œil pour obtenir une pluralité d'images de fond d'œil normalisées ;

un module de filtrage préliminaire (330), conçu pour réaliser un filtrage préliminaire sur une qualité de chacune des images de fond d'œil normalisées pour obtenir une pluralité d'images de fond d'œil qualifiées ;

un module de préparation de données (340), conçu pour préparer un ensemble d'images de fond d'œil cible (200), l'ensemble d'images de fond d'œil cible (200) comporte un ensemble de données à calibrer (210) comportant la pluralité d'images de fond d'œil qualifiées, un ensemble de données d'étalon-or (220) comportant un premier nombre prédéfini d'images de fond d'œil d'étalon-or avec un résultat d'annotation correct connu, et un ensemble de données de détermination d'autocohérence (230) composé d'au moins une image dans l'ensemble de données à calibrer (210), chaque image de l'ensemble d'images de fond d'œil cible (200) est prise en tant que chaque image de fond d'œil cible ;

un module d'annotation (350), conçu pour acquérir une pluralité de groupes de résultats d'annotation de médecin (130) par une pluralité de premiers médecins d'annotation (A) annotant respectivement chaque image dans l'ensemble d'images de fond d'œil cible (200), les résultats d'annotation de médecin (130) comportent au moins un résultat de détermination, le résultat de la détermination comporte au moins des informations de maladie d'absence d'anomalie évidente ou d'une maladie ; et

un module d'évaluation (360), conçu pour calculer une autocohérence et une cohérence d'étalon-or d'un premier médecin d'annotation (A) correspondant en fonction du résultat d'annotation de médecin (130) de manière à obtenir le résultat d'annotation de médecin (130) du premier médecin d'annotation (A) satisfaisant à une condition prédéfinie en tant que résultat d'annotation cible (140), l'autocohérence est obtenue par la prise de l'un quelconque des deux groupes de résultats d'annotation du résultat d'annotation de médecin (130) de chaque image dans l'ensemble de données de détermination d'autocohérence (230) et du résultat d'annotation de médecin (130) d'une image, qui est répétée avec l'image respective dans l'ensemble de données de détermination d'autocohérence (230), dans l'ensemble de données à calibrer (210) en tant que premier groupe de résultats d'annotation et l'autre groupe en tant que second groupe de résultats d'annotation, et la réalisation d'une évaluation à l'aide d'un procédé de détermination et d'évaluation d'autocohérence, la cohérence d'étalon-or est obtenue par la prise du résultat d'annotation correct de l'ensemble de données d'étalon-or (220) en tant que premier groupe de résultats d'annotation et du résultat d'annotation de médecin (130) de chaque image dans l'ensemble de données d'étalon-or (220) en tant que second groupe de résultats d'annotation et utilisation d'un procédé de détermination et d'évaluation de cohérence d'étalon-or ;

un module de rassemblement (370), conçu pour rassembler la pluralité de groupes de résultats d'annotation cibles (140) pour obtenir un résultat d'annotation final (150).

100

110

120

A

A1

A2

A3

| doctor annotation results | doctor annotation results | doctor annotation results | 130 |

| target annotation results | target annotation results | target annotation results | 140 |

B

final annotation results

150

FIG. 1

start

acquiring a plurality of fundus images — S110

performing standardization processing on each fundus image to obtain a plurality of standardized fundus images — S120

performing preliminary filtering on quality of each standardized fundus image to obtain a plurality of qualified fundus images — S130

preparing a target fundus image set including a data set to be calibrated, a gold-standard data set and a self-consistency determination data set — S140

respectively annotating each image in the target fundus image set by a plurality of target annotation doctors to obtain a plurality of groups of doctor annotation results — S150

acquiring a plurality of groups of target annotation results on the basis of a plurality of groups of doctor annotation results meeting a preset condition — S160

gathering the plurality of target annotation results to obtain final annotation results — S170

End

FIG. 2

target fundus image set <u>200</u>

data set to be calibrated <u>210</u>

gold-standard data set <u>220</u>

self-consistency determination data set <u>230</u>

FIG. 3

start

acquiring target self-consistency of annotation doctors with different threshold value

S161

calculating self-consistency mean value μ0 and self-consistency variance σ0

S162

calculating the self-consistency threshold value based on the self-consistency mean value μ0 and the self-consistency variance σ0

S163

end

FIG. 4

FIG. 5

Axis label (y): target self-consistency

Axis label (x): target gold-standard consistency

Legend:
- doctors with experience of 1 to 4 years
- doctors with experience of 5 to 9 years
- doctors with experience of no less than 10 years

Quadrant labels: D1, D2, D3, D4

FIG. 6

start

acquiring each target fundus image — S171

comparing each annotation result of the target fundus image in the plurality of groups of target annotation results — S172

whether the various annotation results are consistent or not — S173

— consistent → taking the annotation result as the final annotation result of the target fundus image — S174

— inconsistent →

the same determination result being included at the same time and only one annotation result including the determination result which is not identified in other annotation results — S175

— yes → annotating the target fundus image as the fundus image to be quality-controlled — S176

performing quality control on the fundus image to be quality-controlled and obtaining the final annotation result — S177

— no → annotating the target fundus image as a difficult fundus image — S178

annotating and arbitrating difficult fundus images to obtain the final annotation result — S179

end

**EP 4 258 205 B1**

## REFERENCES CITED IN THE DESCRIPTION

**Patent documents cited in the description**

- CN 110991486 A
- US 2018165850 A1
- CN 111080577 A